# FASCICULE DE BREVET EUROPEEN

(11) **EP 2 041 133 B1**
(45) Date de publication et mention de la délivrance du brevet: **13.10.2010**
(21) Numéro de dépôt: 07803833.8
(22) Date de dépôt: 03.07.2007
(51) Int. Cl.: C07D 471/04, A61K 31/437, A61P 25/00

(54) **DÉRIVÉS D'IMIDAZO[1,2-A]PYRIDINE-2-CARBOXAMIDES LEUR PRÉPARATION ET LEUR APPLICATION EN THÉRAPEUTIQUE**
IMIDAZO [1,2-A]PYRIDIN-2-CARBOXAMID-DERIVATE, HERSTELLUNGSVERFAHREN UND IHRE VERWENDUNG FÜR THERAPEUTIKA
DERIVATIVES OF IMIDAZO [1,2-A]PYRIDINE-2-CARBOXAMIDES, PREPARATION METHOD THEREOF AND USE OF SAME IN THERAPEUTICS

(30) Priorité: 03.07.2006 FR 0606012
(43) Date de publication de la demande: 01.04.2009
(73) Titulaire: Sanofi-Aventis, 75013 Paris (FR)
(72) Inventeur: EL-AHMAD, Youssef, 92160 Antony (FR); OLIVIER, Anne, 92160 Antony (FR); PEYRONEL, Jean-François, 92160 Antony (FR)
(74) Mandataire: Rauline, Mathilde
(86) Numéro de dépôt international: PCT/FR2007/001125
(87) Numéro de publication internationale: WO 2008/003856

(56) Documents cités:
- WO-A-2006/024834
- WO-A-2006/067446
- WO-A2-2005/048948
- WO-A2-2006/067445
- FR-A1- 2 638 161

## Description

La présente invention se rapporte à des dérivés d'imidazo[1,2*-a*]pyridine-2-carboxamides, à leur préparation et à leur application en thérapeutique dans le traitement ou la prévention de maladies impliquant les récepteurs nucléaires Nurr-1 aussi appelés NR4A2, NOT, TINUR, RNR-1, et HZF3.

La présente invention a pour objet les composés répondant à la formule (I) répondant à la formule (I) : 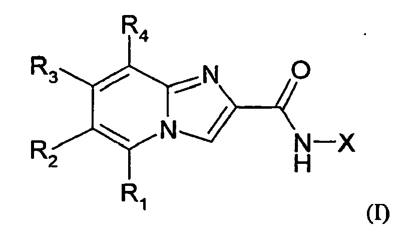 dans laquelle :
- X: représente l'un des groupes suivants :
· un groupe phényle éventuellement substitué par un ou plusieurs groupes choisis indépendamment les uns des autres parmi les atomes ou groupes suivants : halogène, (C₁- C₆)alcoxy, (C₁-C₆)alkyle, (C₃-C₇)cycloalkyle(C₁-C₆)alkyle, (C₃-C₇)cycloalkyle(C₁- C₆)alcoxy, NRaRb,
- R₁: représente un atome d'hydrogène, un halogène, un groupe (C₁-C₆)alcoxy, un groupe (C₁- C₆)alkyle, un groupe (C₃-C₇)cycloalkyle(C₁-C₆)alkyle, un groupe (C₃-C₇)cycloalkyle(C₁- C₆)alcoxy, un amino, un groupe NRcRd ; les groupes alkyles et alcoxy pouvant éventuellement être substitués par un ou plusieurs halogène, hydroxy, amino, ou groupe (C₁-C₆)alcoxy,
- R₂: représente l'un des groupes suivants :
· un atome d'hydrogène,
· un groupe (C₁-C₆)alkyle éventuellement substitué par un ou plusieurs groupes choisis indépendamment les uns des autres parmi un hydroxy, un halogène, un amino, un groupe NRaRb, un groupe phényle
. un groupe (C₁-C₆)alcoxy éventuellement substitué par un ou plusieurs groupes choisis indépendamment les uns des autres parmi hydroxy, halogène, amino, groupe NRaRb,
· un groupe (C₃-C₇)cycloalkyle(C₁-C₆)alkyle,
· un groupe (C₃-C₇)cycloalkyle(C₁-C₆)alcoxy,
· un groupe (C₂-C₆)alcényle,
· un groupe (C₂-C₆)alcynyle,
· un groupe -CO-R₅
· un groupe -CO-NR₆R₇
· un groupe -CO-O-R₈
· un groupe -NR₉-CO-R₁₀
· un groupe -NR₁₁R₁₂,
· un atome d'halogène,
· un groupe cyano,
· un groupe phényle éventuellement substitué par un ou plusieurs groupes choisis indépendamment les uns des autres parmi les atomes ou groupes suivants : halogène, (C₁- C₆)alcoxy, NRaRb, -CO-R₅, -CO-NR₆R₇, -CO-O-R₈, (C₃-C₇)cycloalkyle(C₁-C₆)alkyle, (C₃-C₇)cycloalkyle(C₁-C₆)alcoxy, un groupe (C₁-C₆)alkyle éventuellement substitué par un ou plusieurs hydroxy ou NRaRb,
- R₃: représente un atome d'hydrogène, un groupe (C₁-C₆)alkyle, un groupe (C₁-C₆)alcoxy ou un atome d'halogène,
- R₄: représente un atome d'hydrogène, un groupe (C₁-C₄)alkyle, un groupe (C₁-C₄)alcoxy ou un atome de fluor,
- R₅: représente un atome d'hydrogène, un groupe phényle ou un groupe (C₁-C₆)alkyle,
- R₆ et R₇,: identiques ou différents, représentent un atome d'hydrogène ou un groupe (C₁-C₆)alkyle ou forment avec l'atome d'azote un cycle de 4 à 7 chaînons incluant éventuellement un autre hétéroatome choisi parmi N, O ou S,
- R₈: représente un groupe (C₁-C₆)alkyle,
- R₉ et R₁₀,: ou différents, représentent un atome d'hydrogène ou un groupe (C₁-C₆)alkyle,
- R₁₁ et R₁₂,: identiques ou différents, représentent un groupe (C₁-C₆)alkyle ou forment avec l'atome d'azote un cycle de 4 à 7 chaînons incluant éventuellement un autre hétéroatome choisi parmi N, O ou S,
- Ra et Rb: sont indépendamment l'un de l'autre, hydrogène, (C₁-C₆)alkyle ou forment avec l'atome d'azote un cycle de 4 à 7 chaînons,
- Rc: est hydrogène et Rd est (C₁-C₆)alkyle
et l'un au moins des substituants R₁, R₂, R₃ et R₄ est différent d'hydrogène ;
et quand R₃ est un méthyle, X est non substitué ;
quand R₁ est un méthyle, X est non substitué
quand R₂ est chlore, X n'est pas un para-fluoro-phényle
à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un atome d'halogène : un fluor, un chlore, un brome ou un iode ;
- un groupe alkyle : un groupe aliphatique saturé linéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle etc ;
- un groupe alcényle : un groupe aliphatique mono- ou poly-insaturé, linéaire ou ramifié, comprenant par exemple une ou deux insaturations éthyléniques ;
- un groupe alcoxy : un radical -O-alkyle où le groupe alkyle est tel que précédemment défini ;
- un groupe alcynyle : un groupe aliphatique mono- ou poly-insaturé, linéaire ou ramifié, comprenant par exemple une ou deux insaturations acétylèniques ;

Parmi les composés de formule (I) objets de l'invention, un premier groupe de composés est constitué des composés pour lesquels X est un groupe phényle.

Parmi les composés de formule (I) objets de l'invention, un second groupe de composés est constitué des composés pour lesquels R₁, R₃ et R₄ sont des atomes d'hydrogène.

Parmi les composés de formule (I) objets de l'invention, on peut notamment citer les composés suivants :
6-Chloro*-N-*phénylimidazo[1,2*-a*]pyridine-2-carboxamide
8-Méthyl-*N*-phénylimidazo[1,2*-a*]pyridine-2-carboxamide
6-(Diméthylamino)*-N-*phénylimidazo[1,2*-a*]pyridine-2-carboxamide
6-(1-Hydroxy-1-méthyléthyl)*-N-*phénylimidazo[1,2*-a*]pyridine-2-carboxamide
*N-*(4-Fluorophényl)-6-isopropénylimidazo[1,2*-a*]pyridine-2-carboxamide
6-Chloro*-N-*(2-chlorophényl)imidazo[1*,2-a*]pyridine-2-carboxamide
*N,*6-Diphénylimidazo[1,2*-a*]pyridine-2-carboxamide
*N-*Phényl-6-vinylimidazo[1,2-*a*]pyridine-2-carboxamide
6-Ethyl*-N-*phénylimidazo[1,2*-a*]pyridine-2-carboxamide
6-Formyl*-N-*phénylimidazo[1*,2-a*]pyridine-2-carboxamide
6-Ethynyl*-N-*phénylimidazo[1*,2-a*]pyridine-2-carboxamide
6-[3-(1-Hydroxy-1-méthyléthyl)phényl]*-N-*phénylimidazo[1,2*-a*]pyridine-2-carboxamide
6-[Hydroxy(phényl)méthyl]*-N-*phénylimidazo[1,2*-a*]pyridine-2-carboxamide
Chlorhydrate (1:1) de 6-acétyl*-N-*phénylimidazo[1,2*-a*]pyridine-2-carboxamide
6-Isopropyl*-N-*phénylimidazo[1,2*-a*]pyridine-2-carboxamide
6-(1-Hydroxyéthyl)*-N-*phénylimidazo[1,2*-a*]pyridine-2-carboxamide
6-Acétamido*-N-*phénylimidazo[1,2*-a*] pyridine-2-carboxamide
6-(Diméthylamino)-5-méthyl*-N-*phénylimidazo[1,2*-a*]pyridine-2-carboxamide
6-Méthyl*-N-*phénylimidazo[1,2*-a*]pyridine-2-carboxamide
5-Méthyl*-N-*phénylimidazo[1,2*-a*]pyridine-2-carboxamide
7-Méthyl*-N-*phénylimidazo[1,2*-a*]pyridine-2-carboxamide
6-Bromo*-N-*phénylimidazo[1,2*-a*]pyridine-2-carboxamide
6-Fluoro*-N-*phénylimidazo[1,2*-a*]pyridine-2-carboxamide
6,8-Difluoro*-N-*phénylimidazo[1,2*-a*]pyridine-2-carboxamide
6-Bromo-5-méthyl*-N-*phénylimidazo[1*,2-a*]pyridine-2-carboxamide
6-Iodo*-N-*phénylimidazo[1,2*-a*]pyridine-2-carboxamide
6-Cyano*-N-*phénylimidazo[1,2*-a*]pyridine-2-carboxamide
6-(Hydroxyméthyl)*-N-*phénylimidazo[1,2*-a*]pyridine-2-carboxamide
6-Méthoxy*-N-*phénylimidazo[1,2*-a*]pyridine-2-carboxamide
*N-*(4-fluorophényl)-6-(1-hydroxy-1-méthyléthyl)imidazo[1,2*-a*]pyridine-2-carboxamide
6-Benzoyl*-N-*phénylimidazo[1,2*-a*]pyridine-2-carboxamide
6-Isopropényl*-N-*phénylimidazo[1,2*-a*]pyridine-2-carboxamide
6-Chloro*-N-*(3-fluorophényl)imidazo[1,2*-a*]pyridine-2-carboxamide
6-Chloro*-N-*(3-chlorophényl)imidazo[1,2*-a*]pyridine-2-carboxamide
6-Chloro*-N-*(3-méthoxyphényl)imidazo[1,2*-a*]pyridine-2-carboxamide
6-Chloro*-N-*[4-(diméthylamino)phényl]imidazo[1,2*-a*]pyridine-2-carboxamide
6-Chloro*-N-*(4-chlorophényl)imidazo[1,2*-a*]pyridine-2-carboxamide
6-[2-(Hydroxyméthyl)phényl]*-N-*phénylimidazo[1,2*-a*]pyridine-2-carboxamide
6-[3-(Hydroayméthyl)phényl]*-N-*phénylimidazo[1,2*-a*]pyridine-2-carboxamide
6-[4-(Hydroxyméthyl)phényl]*-N-*phénylimidazo[1,2*-a*]pyridine-2-carboxamide
6-(2-Formylphényl)*-N-*phénylimidazo[1,2*-a*]pyridine-2-carboxamide
6-(3-Formylphényl)*-N-*phénylimidazo[1,2*-a*]pyridine-2-carboxamide
5,6-Diméthyl*-N-*phénylimidazo[1,2*-a*]pyridine-2-carboxamide
3-[2-(Anilinocarbonyl)imidazo[1,2*-a*]pyridin-6-yl]benzoate de méthyle
6-(3-Acétylphényl)*-N-*phénylimidazo[1,2*-a*]pyridine-2-carboxamide
6-(3-Fluorophényl)*-N-*phénylimidazo[1,2*-a*]pyridine-2-carboxamide
6-(4-Méthylphényl)*-N-*phénylimidazo[1,2*-a*]pyridine-2-carboxamide
6-(3-Méthoxypliényl)*-N-*phénylimidazo[1,2*-a*]pyridine-2-carboxamide
6-[3-(Aminométhyl)phényl]*-N-*phénylimidazo[1,2*-a*]pyridine-2-carboxamide
6-(3-Chlorophényl)*-N-*phénylimidazo[1,2*-a*]pyridine-2-carboxamide
6-(3 -Carbamoylphényl)*-N-*phénylimidazo[1*,2-a*]pyridine-2-carboxamide
6-[3-(1-Hydroxyéthyl)phényl]*-N-*phénylimidazo[1,2*-a*]pyridine-2-carboxamide
6-(3-Méthylphényl)*-N-*phénylimidazo[1,2*-a*]pyridine-2-carboxamide
6-(Diéthylamino)*-N-*phénylimidazo[1,2*-a*]pyridine-2-carboxamide et son chlorhydrate (1:1)
6-[3-(Méthylcarbamoyl)phényl]*-N-*phénylimidazo[1*,2-a*]pyridine-2-carboxamide et son chlorhydrate (1:1)
6-Carbamoyl*-N-*phénylimidazo[1*,2-a*]pyridine-2-carboxamide
6-(Diméthylamino)*-N-*(3-fluorophényl)imidazo[1,2-a]pyridine-2-carboxamide
*N-*(2,5-difluorophényl)-6-(diméthylamino)imidazo[1,2-a]pyridine-2-carboxamide
*N*-(2,3-difluorophényl)-6-(diméthylamino)imidazo[1,2-a]pyridine-2-carboxamide
6-(Diméthylamino)*-N-*(2-fluorophényl)imidazo[1,2-a]pyridine-2-carboxamide
*N-*(3-fluorophényl)-6-[3-(hydroxyméthyl)phényl]imidazo[1,2-a]pyridine-2-carboxamide
*N-*(3,5-difluorophényl)-6-[3-(hydroxyméthyl)phényl]imidazo[1,2-a]pyridine-2-carboxamide
*N-*(2-chlorophényl)-6-[3-(hydroxyméthyl)phényl]imidazo[1,2-a]pyridine-2-carboxamide
6-[3-(hydroxyméthyl)phényl]*-N-*(3-méthylphényl)imidazo[1,2-a]pyridine-2-carboxamide
*N-*(2,5-difluorophényl)-6-[3-(hydroxyméthyl)phényl]imidazo[1,2-a]pyridine-2-carboxamide
*N-*(2,3-difluorophényl)-6-[3-(hydroxyméthyl)phenyl]imidazo[1,2-a]pyridine-2-carboxamide
*N-*(2-fluorophényl)-6-[3-(hydroxyméthyl)phényl]imidazo[1,2-a]pyridine-2-carboxamide
*N-*(5-chloro-2-fluorophényl)-6-[3-(hydroxyméthyl)phényl]imidazo[1,2-a]pyridine-2-carboxamide
6-Morpholin-4-yl*-N-*phénylimidazo[1,2*-a*]pyridine-2-carboxamide et son chlorhydrate (1:1)
6-Azétidin-1-yl*-N-*phénylimidazo[1,2*-a*]pyridine-2-carboxamide
6-Iodo-5-méthyl*-N-*phénylimidazo[1,2*-a*]pyridine-2-carboxamide
*N-*(3,5-difluorophényl)-6-iodoimidazo[1,2-a]pyridine-2-carboxamide
*N-*(3-chlorophényl)-6-[3-(hydroxyméthyl)phényl]imidazo[1,2-a]pyridine-2-carboxamide
*N-*(3,5-difluorophényl)-6-(diméthylamino)imidazo[1,2-a]pyridine-2-carboxamide
*N-*(2-chlorophényl)-6-(diméthylamino)imidazo[1,2-a]pyridine-2-carboxamide
6-(Diméthylamino)*-N-*[3-(trifluorométhoxy)phényl]imidazo[1,2-a]pyridine-2-carboxamide
*N-*(3-chloro-2-fluorophényl)-6-[3-(hydroxyméthyl)phényl]imidazo[1,2-a]pyridine-2-carboxamide
6-(Diméthylamino)*-N-*(3-méthylphényl)imidazo[1,2-a]pyridine-2-carboxamide
*N-*(3-chlorophényl)-6-(diméthylamino)imidazo[1,2-a]pyridine-2-carboxamide
*N-*(5-chloro-2-fluorophényl)-6-(diméthylamino)imidazo[1,2-a]pyridine-2-carboxamide
*N-*(3-chloro-2-fluorophényl)-6-(diméthylamino)imidazo[1,2-a]pyridine-2-carboxamide
*N-*[3-(difluorométhoxy)phényl]-6-(diméthylamino)imidazo[1,2-a]pyridine-2-carboxamide
6-(Diméthylamino)*-N-*[3-(trifluorométhyl)phényl]imidazo[1,2-a]pyridine-2-carboxamide

Conformément à l'invention, on peut préparer les composés de formule générale (I) selon le procédé décrit dans schéma 1.

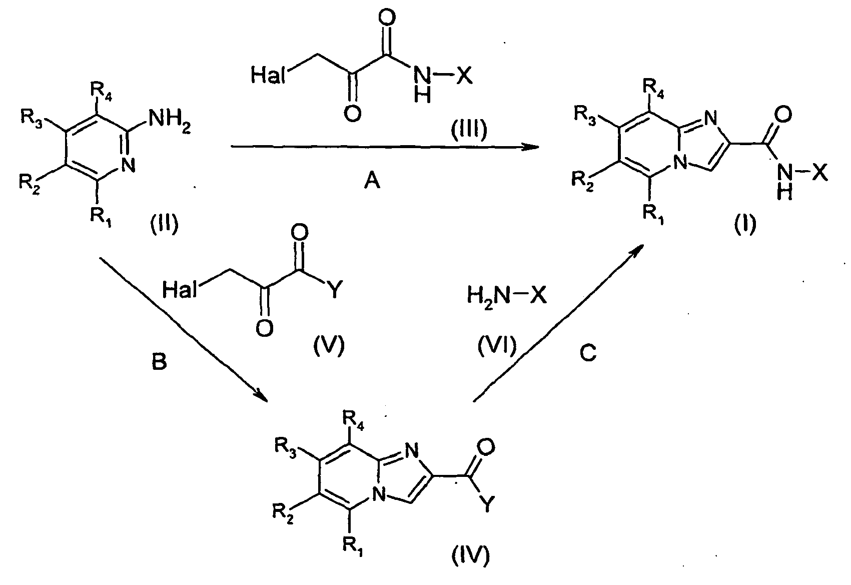

La voie A consiste à préparer les 2-amino-pyridines de formule (II) selon les méthodes connues de l'homme du métier et à former le cycle imidazo [1,2*-a*]pyridine par condensation sur un dérivé de 2-oxo*-N-*aryl-propionamide (III) dans lequel Hal représente un atome de chlore, de brome ou d'iode et X est défini comme précédemment par analogie avec les méthodes décrites par J-J. Bourguignon et coll. dans Aust. J. Chem.1997, 50, 719-725 et par J.G. Lombardino, J. Org. Chem. (1965), 30(7), 2403 par exemple. Les dérivés halogènés de 2-oxo*-N-*aryl-propionamide (III) peuvent être obtenus selon la méthode décrite par R. Kluger et coll. dans J. Am. Chem. Soc., (1984) 106(14), 4017.

La seconde voie de synthèse B, C consiste à coupler un acide imidazopyridine-2-carboxylique ou l'un de ses dérivés, de formule (IV) dans laquelle Y est OH, ou halogène ou C₁-C₆)alcoxy avec une arylamine X-NH2 (VI) dans laquelle X est défini comme précédemment selon des méthodes connues de l'homme du métier. Ainsi l'acide peut être préalablement converti en de ses dérivés réactifs tel que halogènure d'acide, anhydride, anhydride mixte ou ester activé puis mis en réaction avec l'amine (VI) en présence d'une base telle que la diisopropyléthylamine, la triéthylamine ou la pyridine, dans un solvant inerte tel que le THF, le DMF ou le dichlorométhane. Le couplage peut également être réalisé en présence d'un agent de couplage tel que CDI, EDCI, HATU ou HBTU dans les mêmes conditions sans isoler d'intermédiaire réactif. Alternativement on peut faire réagir l'amine (VI) avec un ester de l'acide de formule (IV) en présence d'un catalyseur tel que le triméthylaluminium selon la méthode de Weinreb, S. et coll (Tet. Lett. (1977), 18, 4171) ou le terbutylate de zirconium. Les acides imidazopyridine-2-carboxyliques et leurs dérivés de formule (IV) peuvent être obtenus en condensant les 2-aminopyridines appropriées sur un ester de l'acide 3-halogèno-2-oxo-propionique selon la méthode décrite par J.G. Lombardino dans J. Org. Chem., 30(7), 2403 (1965), puis en déprotégeant l'ester en acide et convertissant le cas échéant l'acide en l'un de ses dérivés.

Les produits de formule (I), et leurs précurseurs de formule (II) ou (IV), peuvent être soumis, si désiré et si nécessaire, pour obtenir des produits de formule (I) ou être tranformés en d'autres produits de formule (I) à l'une ou plusieurs des réactions de transformations suivantes, dans un ordre quelconque :
a) une réaction d'estérification ou d'amidification de fonction acide,
b) une réaction d'amidification de fonction amine,
c) une réaction d'hydrolyse de fonction ester en fonction acide,
d) une réaction de transformation de fonction hydroxyle en fonction alcoxy,
e) une réaction d'oxydation de fonction alcool en fonction aldéhyde ou cétone,
f) une réaction de transformation des fonctions aldéhyde ou cétone en fonction alcool par réduction ou action d'un organométallique tel qu'un organomagnésien,
g) une réaction de transformation de radical nitrile en fonction aldéhyde,
h) une réaction de transformation de radical nitrile en fonction cétone,
i) une réaction d'oxydation de groupe alcènyle en fonction aldéhyde ou cétone,
j) une réaction d'oléfmation de fonction aldéhyde ou cétone en groupe alcènyle,
k) une réaction de deshydratation de groupe hydroxyalkyle en groupe alcényle,
l) une réaction d'hydrogénation totale ou partielle de groupe alcényle ou alcynyle en groupe alcényle ou alkyle,
m) une réaction de couplage catalytique d'un dérivé halogèné et d'un dérivé organométallique tel qu'un dérivé du bore, de l'étain ou du silicium pour introduire un substituant alkyle, alcènyle, alcynyles ou aryle,
n) une réaction de réduction d'un groupe nitro en groupe amino primaire,
o) une réaction de conversion d'un groupe amino primaire ou secondaire en un groupe amino secondaire ou tertiaire par amination réductrice ou alkylation,
p) une réaction de protection des fonctions réactives,
q) une réaction d'élimination des groupements protecteurs que peuvent porter les fonctions réactives protégées,
r) une réaction de salification par un acide minéral ou organique ou par une base pour obtenir le sel correspondant,
s) une réaction de dédoublement des formes racémiques en énantiomères,
lesdits produits de formule (I) ainsi obtenus étant le cas échéant sous toutes les formes isomères possibles racémiques, énantiomères et diastéréoisomères.

Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1 : 6-Chloro-N-phénylimidazo[1,2-a]pyridine-2-carboxamide

A une solution de 0,196 g d'acide 6-chloroimidazo[1,2*-a*]pyridine-2-carboxylique dans 3 mL de dichlorométhane et de 0,146 mL de chlorure de thionyle, on ajoute 1 mL de diméthylformamide. Le mélange réactionnel est agité 3 heures à la température ambiante. On ajoute 0,273 mL d'aniline et agite à la température ambiante pendant 22 heures. On ajoute 20 mL de dichlorométhane et 10 mL d'eau. Après décantation, la phase organique est séchée sur sulfate de magnésium, filtrée, évaporée à sec sous pression réduite, puis purifiée sur une colonne de silice en éluant par du dichlorométhane. Les fractions contenant le produit sont réunies et concentrées à sec sous pression réduite pour donner 0,204 g de 6-chloro*-N-*phénylimidazo[1,2*-a*]pyridine-2-carboxamide sous la forme d'un solide blanc.

### Exemple 2 : 8-Méthyl-N-phénylimidazo[1,2-a]pyridine-2-carboxamide

A une solution de 0,176 g d'acide 8-méthylimidazo[1,2*-a*]pyridine-2-carboxylique dans 12 mL de dichlorométhane, on ajoute 1,67 mL de triéthylamine, 1,53 g de chlorhydrate de 1-(3-diméthylaminopropyl)-3-éthylcarbodiimide et 1,08 g de 1-hydroxy-benzotriazole. Le mélange réactionnel est agité 20 minutes à la température ambiante. On ajoute 0,090 mL d'aniline. Le mélange réactionnel est agité 4 heures à la température ambiante. On ajoute 60 mL de dichlorométhane et 30 mL d'eau. Après décantation, la phase organique est séchée sur sulfate de magnésium, filtrée, évaporée à sec sous pression réduite. Après purification par chromatographie flash (silice, éluant dichlorométhane / acétate d'éthyle 98/02 en volume), on obtient 0,193 g de 8-méthyl*-N-*phénylimidazo[1,2*-a*]pyridine-2-carboxamide sous la forme d'un solide blanc.

### Exemple 3 : 6-(Diméthylamino)-N-phénylimidazo[1,2-a]pyridine-2-carboxamide

A une solution de 60,8 µL d'aniline dans 3 mL de toluène refroidie à 0°, on ajoute goutte à goutte 0,46 mL d'une solution de triméthylaluminium 2M dans le toluène puis à 20°C, 76 mg de 6-diméthylaminoimidazo[1,2*-a*]pyridine-2-carboxylate d'éthyle. Le mélange réactionnel est agité 15 minutes à la température ambiante. On refroidit à 0° puis ajoute 20 mL d'une solution saturée de chlorure d'ammonium. La phase organique est séchée sur sulfate de magnésium, filtrée sur célite, évaporée à sec sous pression réduite. Le résidu est chromatographié sur une cartouche de silice en éluant avec par un mélange de dichlorométhane et d'acétate d'éthyle. Les fractions contenant le produit sont réunies et concentrées à sec sous pression réduite. Le résidu obtenu est cristallisé du méthanol pour donner 36 mg de 6-(diméthylamino)-*N*-phénylimidazo[1,2*-a*]pyridine-2-carboxamide sous la forme d'une poudre grise.

### Exemple 4 : 6-(1-Hydroxy-1-méthyléthyl)-N-phénylimidazo[1,2-a]pyridine-2-carboxamide

Une solution de 100 mg de 2-(6-amino-pyridin-3-yl)-propan-2-ol dans 10 mL de 1,2-diméthoxyéthane est traitée par 190 mg de 3-bromo-2-oxo*-N-*phényl-propionamide puis agitée 15 heures à 25°C et portée au reflux pendant 3 heures. Le mélange réactionnel est concentré à sec sous pression réduite et le résidu repris dans 40 mL d'acétate d'éthyle et 40 mL d'une solution saturée de carbonate de sodium. La phase aqueuse est lavée deux fois par 40 mL d'acétate d'éthyle. Les phases organiques réunies sont séchées et concentrées à sec sous pression réduite. Le résidu est chromatographié sur une cartouche de 40 g de silice en éluant par du dichlorométhane puis par des mélange de dichlorométhane et de méthanol 97/3 puis 95/5. Les fractions contenant le produit attendu sont réunies et concentrées à sec pour donner pour donner 63 mg de 6-(1-hydroxy-1-méthyléthyl)*-N-*phénylimidazo[1,2*-a*]pyridine-2-carboxamide, sous la forme d'un solide blanc.

### Exemple 5 : N-(4-Fluorophényl)-6-isopropénylimidazo[1,2-a]pyridine-2-carboxamide

Une solution de 110 mg de *N-*(4-fluorophényl)-6-(1-hydroxy-1-méthyléthyl)imidazo[1,2-a]pyridine-2-carboxamide et 3,3 mg d'acide paratoluènesulfonique dans 5 mL de xylène est chauffée au reflux pendant 6 heures puis évaporée à sec sous pression réduite. Le résidu est repris par 200 mL de dichlorométhane et 20 mL d'eau. La phase organique est séchée et concentrée et le résidu est purifié par chromatographie sur silice en éluant par un mélange de dichlorométhane et d'acétate d'éthyle (95/5). Les fractions contennat le produit attendu sont réunies et concentrées à sec. Le résidu est trituré et lavé avec de l'éther éthylique, filtré et séché pour donner 68 mg de *N-*(4-fluorophényl)-6-isopropénylimidazo[1,2*-a*]pyridine-2-carboxamide sous la forme d'un solide beige.

### Exemple 6 : 6-Chloro-N-(2-chlorophényl)imidazo[1,2-a]pyridine-2-carboxamide

A une solution de 100 mg d'acide 6-chloroimidazo[1,2*-a*]pyridine-2-carboxylique dans 1 mL de *N,N-*diméthyl-formamide on ajoute 54 µl de 2-chloroaniline, 211 mg de HATU, 75 mg de 1-hydroxy-aminotriazole et 237 µl de *N,N*-diisopropyléthylamine. Le mélange réactionnnel est chauffé à 70°C pendant 16 heures, dilué par une solution saturée d'hydrogenocarbonate de sodium et extrait par de l'acétate d'éthyle. Les phases organiques réunies sont séchées et concentrées sous pression réduite. Le résidu est purifié par chromatographie-flash sur silice en éluant par un mélange hexane/Acétate d'éthyle 70/30 pour donner 62 mg de 6-chloro*-N-*(2-chlorophényl)imidazo[1,2*-a*]pyridine-2-carboxamide sous la forme d'un solide blanc.

### Exemple 7 : N,6-diphénylimidazo[1,2-a]pyridine-2-carboxamide

Dans un tube à microondes on charge 0,391 g de 6-iodo*-N-*phénylimidazo[1,2*-a*]pyridine-2-carboxamide, 0,237 g d'acide phénylboronique, 45 mg de tétrakis(triphénylphosphine)palladium, 4 mL de solution aqueuse 2M de carbonate de sodium, 6 mL d'acétonitrile et 6 mL de toluène. Le mélange est chauffé 20 minutes dans l'appareil à microondes réglé sur 150°C. Après refroidissement, la phase organique est séparée, séchée et évaporée. Le résidu est repris par un mélange de dichlorométhane et de pentane. Le solide est filtré et puis purifié par trituration dans du méthanol pour donner 0,22 g de *N,*6-diphénylimidazo[1,2*-a*]pyridine-2-carboxamide sous la forme d'un solide écru.

### Exemple 8 : N-phényl-6-vinylimidazo[1,2-a]pyridine-2-carboxamide

Un mélange de 0,73 g de 6-iodo*-N-*phénylimidazo[1,2*-a*]pyridine-2-carboxamide, 209 mg de tétrakis(triphénylphosphine)palladium(0), 587 µL de tributylvinylétain et 17 mL de DMF est chauffé 10 minutes à 130°C dans un appareil à microondes puis concentré à sec. Le résidu est repris dans 100 mL d'eau et extrait par deux fois 70 mL d'acétate d'éthyle. Les phases organiques réunies sont lavées par une solution saturée de chlorure de sodium, séchées sur sulfate de magnésium et évaporées à sec sous pression réduite. Le solide est trituré dans de l'acétate d'éthyle essoré et lavé par de l'acétate d'éthyle puis par de l'éther isopropylique, repris dans un mélange de méthanol et de dichlorométhane. L'insoluble est filtre et lavé par du méthanol. Le filtrat est concentré à sec sous pression réduite pour donner 0,29 g de *N-*phényl-6-vinylimidazo[1,2-a]pyridine-2-carboxamide sous la forme d'un solide blanc.

### Exemple 9 : 6-Ethyl-N-phénylimidazo[1,2-a]pyridine-2-carboxamide

Une solution de 60 mg de *N-*phényl-6-vinylimidazo[1,2*-a*]pyridine-2-carboxamide dans 15 mL de méthanol, est hydrogènée pendant 45 minutes à 25°C sous 1 bar d'hydrogène en présence de 24 mg de palladium à 10 % sur charbon. La réaction étant incomplète le produit est recyclé dans les même conditions. Après filtration le mélange réactionnel est concentré à sec sous pression réduite. Le résidu est repris dans 50 mL d'acétate d'éthyle. La phase organique est lavée à l'eau, décantée, séchée et concentrée à sec sous pression réduite pour donner 60 mg de 6-éthyl*-N-*phénylimidazo[1,2*-a*]pyridine-2-carboxamide sous la forme d'un solide blanc

### Exemple 10 : 6-Formyl-N-phénylimidazo[1,2-a]pyiridine-2-carboxamide

Une suspension de 150 mg de *N-*phényl-6-vinylimidazo[1,2*-a*]pyridine-2-carboxamide, 232 µL de tétroxyde d'osmium et 167,5 mg de périodate de sodium dans un mélange de 6 mL de THF, 3 mL de t-butanol et 3 mL d'eau, est agitée pendant 20 heures à 20°C puis encore 48 heures en rajoutant à 4 reprises 100 µL de tétroxyde d'osmium et 80 mg de périodate de sodium. Le mélange réactionnel est versé dans 50 mL d'eau et que l'on extrait deux fois par 50 mL d'acétate d'éthyle. Les phases organiques réunies sont lavée par une solution saturée aqueuse de chlorure de sodium, décantées, séchées et concentrées à sec sous pression réduite. Le résidu est chromatographié sur silice en éluant par un mélange de cyclohexane et d'acétate d'éthyle (gradient de 0 à 50 %) pour donner 100 mg de 6-formyl*-N-*phénylimidazo[1,2*-a*]pyridine-2-carboxamide sous la forme d'un solide blanc

### Exemple 11 : 6-Ethynyl-N-phénylimidazo[1,2-a]pyridine-2-carboxamide

Dans un tube à microondes de 20 mL on charge 0,2 g de 6-iodo*-N-*phénylimidazo[1,2*-a*]pyridine-2-carboxamide, 156µL de triméthylsilylacétylène, 20 mg de dichloro bis(triphénylphosphine)-palladium, et 2 mL de pipéridine. Le mélange est chauffé 15 minutes dans l'appareil à microondes réglé sur 130°C. Après refroidissement, le mélange est versé dans 50 mL de solution aqueuse saturée de chorure d'ammonium. On extrait 2 fois par 70 mL d'éther éthylique. Les phases organiques réunies sont décantées, séchées et concentrées à sec sous pression réduite. Le résidu est repris dans 4 mL d'une solution 1M de fluorure de tétrabutylammonium dans le THF et agité 16 heures à 25 °C. Après évaporation à sec du milieu réactionnel, le résidu est chromatographié sur silice en éluant par un mélange de cyclohexane et d'acétate d'éthyle (gradient de 0 à 35 %) pour donner 30 mg de 6-éthynyl*-N-*phénylimidazo[1,2*-a*]pyridine-2-carboxamide sous la forme d'un solide beige.

### Exemple 12 : 6-[3-(1-Hydroxy-1-méthyléthyl)phényl]-N-phénylimidazo[1,2-a]pyridine-2-carboxamide

A une suspension de 87 mg de 3-[2-(anilinocarbonyl)imidazo[1,2*-a*]pyridin-6-yl]benzoate de méthyle dans 5 mL de THF placée sous atmosphère d'argon et refroidie à 10°C, on ajoute lentement 781 µL d'une solution 3M de chlorure de méthylmagnésium dans le THF. Le mélange est agité 16 heures en laissant la température remonter à 20°C puis versé dans 30 mL de solution aqueuse saturée de chorure d'ammonium. On extrait par 100 mL d'acétate d'éthyle. La phase organique est décantée, séchée et concentrée à sec sous pression réduite. Le résidu est chromatographié sur silice en éluant par un mélange de dichlorométhane et d'acétate d'éthyle (75/25) pour donner 22 mg de 6-[3-(1-hydroxy-1-méthyléthyl)phényl]*-N-*phénylimidazo[1,2-a]pyridine-2-carboxamide sous la forme d'un solide écru.

### Exemple 13 : 6-[Hydroxy(phényl)méthyl]-N-phénylimidazo[1,2-a]pyridine-2-carboxamide

A une suspension de 157 mg de 6-benzoyl*-N-*phénylimidazo[1,2*-a*]pyridine-2-carboxamide dans 10 mL de méthanol refroidie à 0°C on ajoute 17,4 mg de borohydrure de sodium. Le mélange réactionnel est agité 16 heures en laissant la température remonter à 20°C. On ajoute 20 mg de borohydrure de sodium et agite encore 1,5 heure à 20°C. Après évaporation à sec le résidu est repris dans 50 mL d'eau et 200 mL d'acétate d'éthyle. La phase organique est décantée, séchée et concentrée à sec sous pression réduite pour donner 122 mg de 6-[hydroxy(phényl)méthyl]-*N-*phénylimidazo[1,2*-a*]pyridine-2-carboxamide sous la forme d'un solide blanc.

### Exemple 14 : Chlorhydrate (1:1) de 6-acétyl-N-phénylimidazo[1,2-a]pyridine-2-carboxamide

Une solution de 90 mg de 6-(1-éthoxyvinyl)*-N-*phénylimidazo[1,2-o]pyridine-2-carboxamide dans 2 mL de diclorométhane est agitée pendant 18 heures à 20°C avec 1 mL d'acide chlorhydrique 2N. Le solide est filtré, lavé par du dichlorométhane puis de l'éther diisopropylique et séché pour donner 67 mg de chlorhydrate (1:1) de 6-acétyl*-N-*phénylimidazo[1,2*-a*]pyridine-2-carboxamide sous la forme d'un solide blanc.

### Exemple 15 : 6-Isopropyl-N-phénylimidazo[1,2-a]pyridine-2-carboxamide

### 15. 1 6-Isopropyl-imidazo[1,2-a]pyridine-2-carboxylate d'éthyle

Le 6-isopropyl-imidazo[1,2-a]pyridine-2-carboxylate d'éthyle est préparé en condensant la 2-amino 5-isopropyl-pyridine (PCT Int. Appl. WO 2005028444) sur du 3-bromopyruvate d'éthyle selon la méthode décrite par J.G. Lombardino dans J. Org. Chem. (1965), 30(7), 2403
Spectre RMN ¹H (DMSO-d6, δ en ppm) : 1,24 (d, J = 7,0 Hz, 6H) ; 1,32 (t, J = 7,0 Hz, 3H) ; 2,91 (m, 1H) ; 4,30 (q, J = 7,0 Hz, 2H) ; 7,33 (dd, J = 1,5 et 9,5 Hz, 1H) ; 7,54 (d, J = 9,5 Hz, 1H) ; 8,39 (s large, 1H) ; 8,45 (s, 1H).

### 15. 2 6-Isopropyl-N-phénylimidazo[1,2-a]pyridine-2-carboxamide

Une solution de 344 mg de 6-isopropylimidazo[1,2*-a*]pyridine-2-carboxylate d'éthyle, 152 mg d'aniline, 40 mg de 1-hydroxy-7-azabemotriazole (HOAt) et 316 mg de tertbutylate de zirconium dans 5 mL de toluène est agité 16 heures à température ambiante dans un tube à microondes. Le milieu est concentré à sec sous pression réduite et le résidu est chromatographié sur une cartouche de silice en éluant par un mélange de dichlorométhane et d'acétate d'éthyle 50/50. Les fractions contenant le produit attendu sont réunies et concentrées à sec sous pression réduite pour donner 63 mg de 6-isopropyl*-N-*phénylimidazo[1,2-a]pyridine-2-carboxamide sous la forme d'un solide blanc.

### Exemple 16 : 6-[(RS)-1-hydroxy-éthyl]-N-phénylimidazo[1,2-a]pyridine-2-carboxamide

Une suspension de 150 mg de 6-formyl*-N-*phénylimidazo[1,2*-a*]pyridine-2-carboxamide dans 10 mL de tétrahydrofuranne est refroidie à 5°C. On ajoute goutte à goutte 1,9 mL d'une solution 3M de chlorure de méthylmagnésium dans le tétrahydrofuranne. Le mélange réactionnel est agité pendant 4 heures à 5°C puis traité par 20 mL de solution saturée de chlorure d'ammonium agité puis dilué par 30 mL d'acétate d'éthyle. La phase aqueuse est extraite à l'acétate d'éthyle et les phases organiques réunies sont lavées par une solution saturée de chlorure de sodium, séchées et concentrées à sec sous pression réduite. Après chromatographie sur silice en éluant par du dichlorométhane puis par des mélanges de dichlorométhane et d'acétate d'éthyle 85/15 puis 60/40, les fractions contenant le produit attendu sont réunies et évaporées à sec sous pression réduite pour donner 59 mg de 6-[(RS)-1-hydroxy-éthyl]*-N-*phénylimidazo[1,2*-a*]pyridine-2-carboxamide sous la forme d'un solide blanc.

### Exemple 17: 6-Acétamido-N-phénylimidazo[1,2-a]pyridine-2-carboxamide

### 65. 1 6-Acétamidoimidazo[1,2-a]pyridine-2-carboxylate d'éthyle

Une suspension de 0,2 g de 6-aminoimidazo[1,2-a]pyridine-2-carboxylate d'éthyle (Heterocycles 38(7), 1527 (1994)) dans 1 mL d'anhydride acétique est chauffée au reflux pendant 45 minutes. Le mélange réactionnel est concentré sous pression réduite ; le résidu est repris par de l'eau et alcalinisé par addition d'une solution 1N de soude. Le solide est filtré et séché pour donner 150 mg de 6-acétamidoimidazo[1,2-a]pyridine-2-carboxylate d'éthyle sous la forme d'un solide écru Spectre RMN ¹H (DMSO-d6, δ en ppm) : 1,31 (t, J = 7,0 Hz, 3 H) ; 2,09 (s, 3H) ; 4,30 (q, J = 7,0 Hz, 2H) ; 7,24 (dd, J = 1,5 et 9,5 Hz, 1H) ; 7,59 (d, J = 9,5 Hz, 1H) ; 8,60 (s, 1H) ; 9,23 (s large, 1H) ; 10,1 (s, 1H).
Spectre de masse (IE) : m/z= 246 [M-H]⁻ , m/z = 248 [M+H]⁺.

### 65. 2 6-Acétamido-N-phénylimidazo[1,2-a]pyridine-2-carboxamide

En opérant de manière analogue à l'exemple 3, on obtient à partir de 150 mg de 6-acétamidoimidazo[1,2-a]pyridine-2-carboxylate d'éthyle, 53 mg de 6-acétamido*-N-*phénylimidazo[1,2-a]pyridine-2-carboxamide) sous la forme d'un solide écru.

### Exemple 18: 6-Diméthylamino-5-méthyl-N-phénylimidazo[1,2-a]pyridine-2-carboxamide

### 69. 1 6-Diméthylamino-5-méthylimidazo[1,2-a]pyridine-2-carboxylate d'éthyle

A une solution de 235 mg de 6-amino-5-méthylimidazo[1,2-a]pyridine-2-carboxylate d'éthyle (Heterocycles 38(7), 1527 (1994)) dans 4 mL d'acide formique on ajoute 1 mL de solution aqueuse de formaldéhyde (formol) puis on chauffe dans un bain à 100°C pendant 4 heures. Après refroidissement, le mélange réactionnel est neutralisé par addition d'une solution 5N de soude et extrait par de l'acétate d'éthyle. La phase organique est séchée et évaporée à sec sous pression réduite. Le résidu est chromatographié sur une cartouche de silice en éluant par un mélange de dichlorométhane et d'acétate d'éthyle 75/52. Les fractions contenant le produit attendu sont réunies et évaporées à sec sous pression réduite pour donner 113 mg de 6-diméthylamino-5-méthylimidazo[1,2-a]pyridine-2-carboxylate d'éthyle sous la forme d'un solide écru.
Spectre RMN ¹H (DMSO-d6, δ en ppm) : 1,32 (t, J = 7,0 Hz, 3H) ; 2,61 (s, 3H) ; 2,64 (s, 6H) ; 4,32 (q, J = 7,0 Hz, 2H) ; 7,49 (d, J = 9,5 Hz, 1H) ; 7,52 (d, J=9,5 Hz, 1H) ; 8,32 (s, 1H). Spectre de masse (IE) : (LC-MS-DAD-ELSD) m/z = 248 [M+H]⁺, m/z = 220 [MH- C₂H₅]⁺.

### 69. 2 6-Diméthylamino-5-méthyl-N-phénylimidazo[1,2-a]pyridine-2-carboxamide

En opérant de manière analogue à l'exemple 3, on obtient à partir de 112 mg de 6-diméthylamino-5-méthylimidazo[1,2-a]pyridine-2-carboxylate d'éthyle, 86 mg de 5-méthyl-6-nitro-*N-*phénylimidazo[1,2-a]pyridine-2-carboxamide sous la forme d'un solide écru.

Les intermédiaires décrits ci-dessous sont utiles à la préparation des composés de la présente invention.

### 2-Amino-pyridines de formule générale (II)

### 2-(6-Amino-pyridin-3-yl)-propan-2-ol

A une solution de 0,7 g de 6-amino-nicotinate de méthyle dans 65 mL de THF, refroidie à 10°C et sous argon, on ajoute goutte à goutte 15 mL de solution 3 M de chlorure de méthylmagnésium dans le THF. Le mélange réactionnel est agité 15 h en laissant remonter la température à 20°C, puis refroidi de nouveau dans un bain de glace. On ajoute lentement 100 mL de solution saturée de chlorure d'ammonium puis 200 mL d'acétate d'éthyle. La phase organique est séchée et concentrée à sec. Le résidu est repris dans de l'acétate d'thyle. Le précipité est essoré et séché pour donner 0,4 g de 2-(6-amino-pyridin-3-yl)-propan-2-ol sous la forme d'un solide jaune pâle.
Spectre RMN ¹H (DMSO-d6, δ en ppm) : 1,36 (s, 6H) ; 4,82 (s, 1H) ; 5,67 (s large, 2H) ; 6,37 (d, J = 9,0 Hz, 1H) ; 7,42 (dd, J = 2,5 et 9,0 Hz, 1H) ; 7,98 (d, J = 2,5 Hz, 1H)
Spectre de masse (IE) : m/z 152 : [M⁺], m/z 137 : [M⁺]-CH₃ (pic de base)

### 5-Diméthylaminopyridine-2-amine

### Diméthyl-6-nitro-pyridine-3-amine

A une solution de 1 g de 5-bromo-2-nitro-pyridine dans 5 mL d'éthanol on ajoute 6 mL de solution 2 M de diméthylamine dans le tétrahydrofuranne. Le mélange réactionnel est chauffé 2 heures à 140°C dans un appareil à microondes. Après refroidissement, le solide formé est séparé et lavé à l'éther éthylique pour donner 850 mg de diméthyl-6-nitro-pyridine-3-amine sous la forme d'un solide jaune.
Spectre RMN ¹H (DMSO-d6, δ en ppm) : 3,12 (s, 6H) ; 7,21 (dd, J = 3,0 et 9,5 Hz, 1H) ; 8,02 (d, J = 3,0 Hz, 1H) ; 8,15 (d, J = 9,5 Hz, 1H)
Spectre de masse (IE) : m/z 167 (pic de base) : [M⁺], m/z 137 : [M⁺]- NO , m/z 121: [M⁺]-NO₂.

### 5-Diméthylaminopyridine-2-amine

La diméthyl-6-nitro-pyridine-3-amine obtenue ci-dessus est reprise dans 25 mL d'éthanol. Après ajout de 4,8 g de chlorure stanneux, le mélange réactionnel est chauffé au reflux pendant 30 minutes puis concentré à sec. Le résidu est chromatographié sur une colonne de silice (40-63 µm) en éluant par un mélange de dichlorométhane et de méthanol ammoniacal 90/10. Les fractions contenant le produit attendu sont réunies et concentrées pour donner 750 mg de 5-diméthylaminopyridine-2-amine sous la forme d'un solide pateux jaune.
Spectre RMN ¹H (DMSO-d6, δ en ppm) : 2,80 (s, 6H) ; 6,94 (d, J = 9,5 Hz, 1H) ; 7,18 (d, J = 3,0 Hz, 1H) ; 7,32 (s large, 2H) ; 7,83 (dd, J = 3,0 et 9,5 Hz, 1H)
Spectre de masse (IE) : m/z 137 (pic de base) : [M⁺], m/z 122 : [M⁺]-CH₃.

### 5-(Azétidin-1-yl)pyridine-2-amine

### 5-Azétidin-1-yl-6-nitro-pyridine

Dans un tube à microondes on charge 1 g de 5-bromo-2-nitro-pyridine, 3,5 g de carbonate de césium, 825 mg de bis(diphénylphosphino)ferrocène, 110 mg d'acétate de palladium et 15 m de toluène puis on ajoute 424 mg d'azétidine. Le tube est agité et chauffé dans un bain à 105°C puis laissé 16 h à température ambiante. Le mélange réactionnel est filtré, le solide rincé par du dichlorométhane. Les filtrats réunis sont concentrés à sec sous pression réduite et le résidu est chromatographié sur une cartouche de silice en éluant par du dichlorométhane. Les fractions contenant le produit attendu sont réunies et concentrées à sec sous pression réduite pour donner 550 mg de 5-azétidin-1-yl-6-nitro-pyridine sous la forme d'un solide jaune.

### 5-(Azétidin-1-yl)pyridine-2-amine

La 5-azétidin-1-yl-6-nitro-pyridine obtenue ci-dessus est reprise dans 10 mL d'éthanol et hydrogènée en présence de 65 mg de palladium à 10 % sur charbon sous une pression de 1 bar et à 30°C. Le mélange réactionnel est filtré, le filtrat dilué par du méthanol ammoniacal 7N puis concentré à sec à 30°C sous pression réduite. Le résidu est repris dans du dichlorométhane, un insoluble est éliminé et le résidu obtenu après évaporation à sec est chromatographié sur une cartouche de silice en éluant par un mélange de dichlorométhane et de méthanol ammoniacal 7N 90/10. Les fractions contenant le produit attendu sont réunies et concentrées pour donner 185 mg de 5-(azétidin-1-yl)pyridine-2-amine sous la forme d'une huile rouge.
Spectre RMN ¹H (DMSO-d6, δ en ppm) : 2,23 (m, 2H) ; 3,65 (t, J = 7,5 Hz, 4H) ; 5,12 (s large, 2H) ; 6,37 (d, J = 9,0 Hz, 1H) ; 6,68 (dd, J = 3,0 et 9,0 Hz, 1H) ; 7,21 (d, J = 3,0 Hz, 1H).
Spectre de masse (IE) : m/z = 226 [M]⁺.

### Dérivés d'acides imidazo[1,2-a]pyridine-2-carboxyliques de formule générale (IV)

### 6-Diméthylamino-imidazo[1,2-a]pyridine-2-carboxylate d'éthyle

A une suspension de 0,2 g de 5-diméthylaminopyridine-2-amine dans 3 mL de DME on ajoute 215 µL de bromopyruvate d'éthyle. Le mélange réactionnel est agité à 20°C pendant 16 heures puis après ajout de 3 mL d'éthanol pendant 16 heures au reflux et enfin concentré sous pression réduite. Le résidu est filtré sur une cartouche de 15 g de silice en éluant par un mélange de dichlorométhane et de méthanol (98/2). Les fractions contenant le produit attendu sont réunies et lavées par par une solution saturée de bicarbonate de sodium. La phase organique est séchée et concentrée à sec sous pression réduite pour donner 76 mg de 6-diméthylamino-imidazo[1,2*-a*]-pyridine-2-carboxylate d'éthyle sous la forme d'une huile verte utilisée telle quelle dans la suite de la synthèse.
En opérant de manière analogue on obtient le **6,8-difluoro-imidazo[1,2*-a*]pyridine-2-carboxylate d'éthyle**
Spectre RMN ¹H (DMSO-d6, δ en ppm) : 1,33 (t, J = 7,5 Hz, 3H) ; 4,36 (q, J = 7,5 Hz, 2H) ; 7,66 (ddd, J = 2,0 - 9,0 et 11,5 Hz, 1H) ; 8,65 (d, J = 3,0 Hz, 1H) ; 8,70 (m, 1H)
Spectre de masse (IE): m/z = 226 [M]⁺, m/z = 181 [M-OC₂H₅]⁺, m/z = 154 [M-C₃H₄O_{z}]⁺ (pic de base).

### 6-(1-Ethoxyvinyl)-N-phénylimidazo[1,2-a]pyridine-2-carboxamide

En opérant de manière analogue à l'exemple 8 en remplaçant le tributylvinylétain par du tributyl(1-éthoxyvinyl)étain, on obtient le 6-(1-éthoxyvinyl)*-N-*phénylimidazo[1,2-a]pyridine-2-carboxamide sous la forme d'un solide beige.
Spectre RMN ¹H (DMSO-d6, δ en ppm) : 1,40 (t, J = 7,0 Hz, 3H) ; 3,97 (q, J = 7,0 Hz, 2H) ; 4,46 (d, J = 3,0 Hz, 1H) ; 4,90 (d, J = 3,0 Hz, 1H) ; 7,09 (t, J = 8,0 Hz, 1H) ; 7,34 (t large, J = 8,0 Hz, 2H) ; 7,61 (d, J = 9,5 Hz, 1H) ; 7,66 (dd, J = 2,0 et 9,5 Hz, 1H) ; 7,89 (d large, J = 8,0 Hz, 2H) ; 8,57 (s, 1H) ; 8,83 (s large, 1H) ; 10,2 (s, 1H).

Les tableaux qui suivent illustrent les structures chimiques (tableau 1) et les caractèristiques spectroscopiques (tableau 2) de quelques exemples de composés selon l'invention.

**Tableau 1**

| | | | | | | |
|---|---|---|---|---|---|---|
| 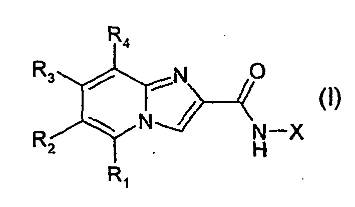 | | | | | | |

| **Ex** | **R₁** | **R₂** | **R₃** | **R₄** | **X** | **sel** |
|---|---|---|---|---|---|---|
| **1** | H | Cl | H | H | Ph | |
| **2** | H | H | H | Me | Ph | |
| **3** | H | ∼NMe₂ | H | H | Ph | |
| **4** | H | ∼CMe₂OH | H | H | Ph | |
| **5** | H | ∼CMe=CH₂ | H | H | 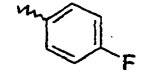 | |
| **6** | H | Cl | H | H | 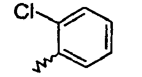 | |
| **7** | H | Ph | H | H | Ph | |
| **8** | H | ∼CH=CH₂ | H | H | Ph | |
| **9** | H | ∼CH₂CH₃ | H | H | Ph | |
| **10** | H | ∼CH=O | H | H | Ph | |
| **11** | H | ∼C≡CH | H | H | Ph | |
| **12** | H | 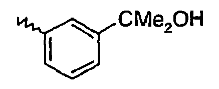 | H | H | Ph | |
| **13** | H | 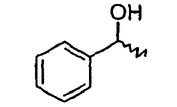 | H | H | Ph | |
| **14** | H | ∼COCH₃ | H | H | Ph | HCl |
| **15** | H | iPr | H | H | Ph | |
| **16** | H | ∼CHOHMe | H | H | Ph | |
| **17** | H | ∼NHCOMe | H | H | Ph | |
| **18** | Me | ∼NMe₂ | H | H | Ph | |
| **19** | H | Me | H | H | Ph | |
| **20** | Me | H | H | H | Ph | |
| **21** | H | H | Me | H | Ph | |
| **22** | H | Br | H | H | Ph | |
| **23** | H | F | H | H | Ph | |
| **24** | H | F | H | F | Ph | |
| **25** | Me | Br | H | H | Ph | |
| **26** | H | I | H | H | Ph | |
| **27** | H | ∼CN | H | H | Ph | |
| **28** | H | ∼CH₂OH | H | H | Ph | |
| **29** | H | ∼OMe | H | H | Ph | |
| **30** | H | ∼CMe₂OH | H | H | 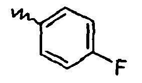 | |
| **31** | H | ∼COPh | H | H | Ph | |
| **32** | H | ∼CMe=CH₂ | H | H | Ph | |
| **33** | H | Cl | H | H | 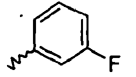 | |
| **34** | H | Cl | H | H | 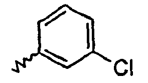 | |
| **35** | H | Cl | H | H | 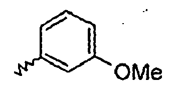 | |
| **36** | H | Cl | H | H | 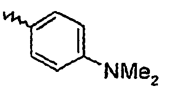 | |
| **37** | H | Cl | H | H | 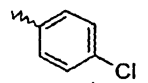 | |
| **38** | H | 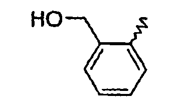 | H | H | Ph | |
| **39** | H | 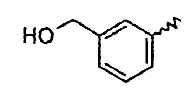 | H | H | Ph | |
| **40** | H | 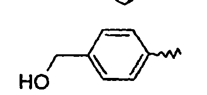 | H | H | Ph | |
| **41** | H | 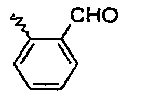 | H | H | Ph | |
| **42** | H | 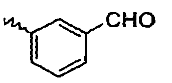 | H | H | Ph | |
| **43** | Me | Me | H | H | Ph | |
| **44** | H | 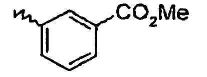 | H | H | Ph | |
| **45** | H | 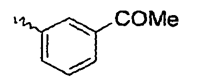 | H | H | Ph | |
| **46** | H | 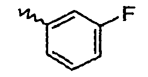 | H | H | Ph | |
| **47** | H | 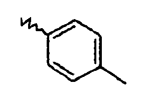 | H | H | Ph | |
| **48** | H | 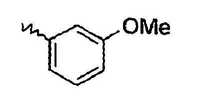 | H | H | Ph | |
| **49** | H | 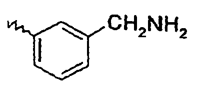 | H | H | Ph | |
| **50** | H | 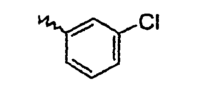 | H | H | Ph | |
| **51** | H | 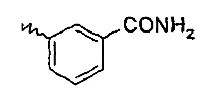 | H | H | Ph | |
| **52** | H | 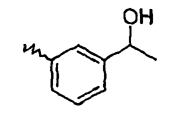 | H | H | Ph | |
| **53** | H | 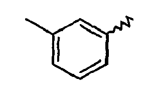 | H | H | Ph | |
| **54** | H | ∼NEt₂ | H | H | Ph | HCl |
| **55** | H | 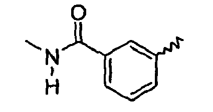 | H | H | Ph | HCl |
| **56** | H | ∼CONH₂ | H | H | Ph | |
| **57** | H | ∼NMe₂ | H | H | 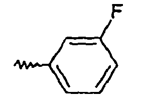 | |
| **58** | H | ∼NMe₂ | H | H | 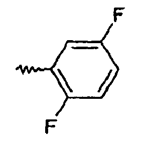 | |
| **59** | H | ∼NMe₂ | H | H | 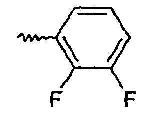 | |
| **60** | H | ∼NMe₂ | H | H | 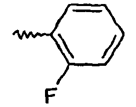 | |
| **61** | H | 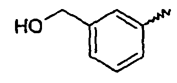 | H | H | 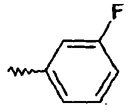 | |
| **62** | H | 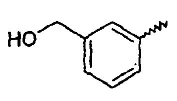 | H | H | 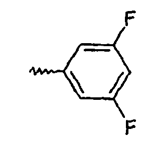 | |
| **63** | H | 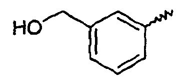 | H | H | 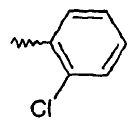 | |
| **64** | H | 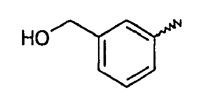 | H | H | 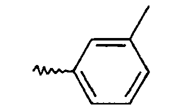 | |
| **65** | H | 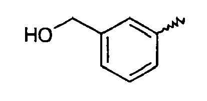 | H | H | 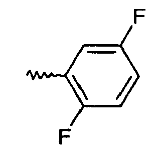 | |
| **66** | H | 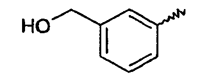 | H | H | 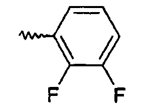 | |
| **67** | H | 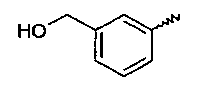 | H | H | 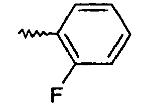 | |
| **68** | H | 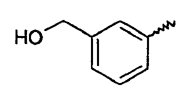 | H | H | 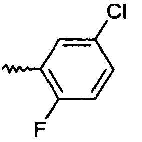 | |
| **69** | H | 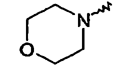 | H | H | Ph | HCl |
| **70** | H | 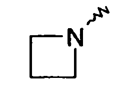 | H | H | Ph | |
| **71** | H | I | Me | H | Ph | |
| **72** | H | I | H | H | 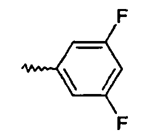 | |
| **73** | H | 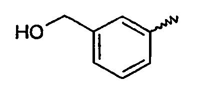 | H | H | 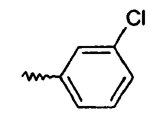 | |
| **74** | H | ∼NMe₂ | H | H | 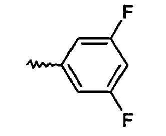 | |
| **75** | H | ∼NMe₂ | H | H | 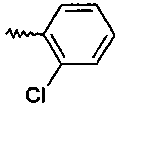 | |
| **76** | H | ∼NMe₂ | H | H | 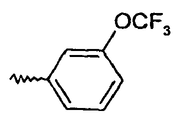 | |
| **77** | H | 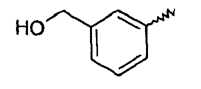 | H | H | 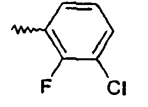 | |
| **78** | H | ∼NMe₂ | H | H | 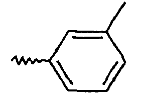 | |
| **79** | H | ∼NMe₂ | H | H | 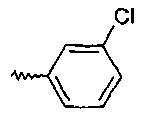 | |
| **80** | H | ∼NMe₂ | H | H | 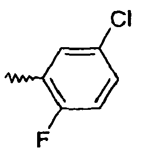 | |
| **81** | H | ∼Me₂ | H | H | 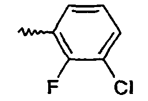 2 | |
| **82** | H | ∼NMe₂ | H | H | 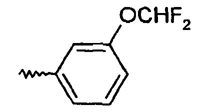 | |
| **83** | H | ∼NMe₂ | H | H | 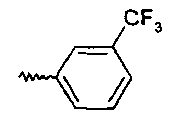 | |

**Tableau 2**

| **Ex** | **Caractérisations** |
|---|---|
| **1** | Spectre RMN ¹H (DMSO-d6, δ en ppm) : 7,10 (t large, J = 7,5 Hz, 1H) ; 7,35 (t large, J = 7,5 Hz, 2H) ; 7,45 (dd, J = 2,0 et 9,5 Hz, 1H) ; 7,70 (d, J = 9,5 Hz, 1H) ; 7,88 (d large, J = 8,0 Hz, 2H) ; 8,48 (s, 1H) ; 8,90 (s large, 1H) ; 10,3 (s large, 1H) Spectre de masse (IE) : m/z 271 : [M⁺], m/z 179 (pic de base) : [M⁺]- NHPh , m/z 243 : [M⁺]-[CO]. |
| **2** | Spectre RMN ¹H (DMSO-d6, δ en ppm) : 2,59 (s, 3H) ; 6,91 (t, J = 7,0 Hz, 1H) ; 7,10 (t large, J = 7,5 Hz, 1H) ; 7,18 (d large, J = 7,0 Hz, 1H) ; 7,35 (t large, J = 7,5 Hz, 2H); 7,86 (d large, J = 8,0 Hz, 2H) ; 8,46 (d large, J = 7,0 Hz, 1H) ; 8,51 (s, 1H) ; 9,96 (s large, 1H) Spectre de masse (IE) : m/z 251 (pic de base) : [M]⁺, m/z 159 : [M-NHPh]⁺, m/z 223 : [M-CO]⁺. |
| **3** | Spectre RMN ¹H (DMSO-d6, δ en ppm) : 2,86 (s, 6H) ; 7,07 (t large, J = 7,5 Hz, 1H) ; 7,33 (t large, J = 7,5 Hz, 2H) ; 7,35 (dd, J = 2,5 et 10,0 Hz, 1H) ; 7,51 (d, J = 10,0 Hz, 1H) ; 7,87 (d large, J = 8,0 Hz, 2H) ; 7,89 (d partiellement masqué, J = 2,5 Hz, 1H) ; 8,33 (s, 1H) ; 10,1 (s, 1H). Spectre de masse (ES) : m/z 281 [M+H]⁺. Spectre IR (KBr) : 3345 ; 3140 ; 2803 ; 1665 ; 1643 ; 1594 ; 1552 ; 1523 ; 1503; 1443 ; 1310 ; 1208 ; 920 ; 798 ; 753 & 692 cm⁻¹. |
| **4** | Spectre RMN ¹H (DMSO-d6, δ en ppm) : 1,49 (s, 6H) ; 5,32 (s, 1H) ; 7,09 (t, J = 7,5 Hz, 1H) ; 7,34 (t, J = 7,5 Hz, 2H) ; 7,49 (dd, J = 2,0 et 9,5 Hz, 1H) ; 7,59 (d, J = 9,5 Hz, 1H) ; 7,89 (d, J = 8,0 Hz, 2H) ; 8,53 (s, 1H) ; 8,63 (s large, 1H) ; 10,2 (s large, 1H). Spectre IR (KBr) : 3363 ; 1666 ; 1597 ; 1559; 1527; 1504 ; 1443 ; 1319 ; 1204 & 757 cm⁻¹ Spectre de masse (IE) : m/z=295 [M]⁺ ; m/z=203 [M-NHPh]⁺ (pic de base) ; m/z=43 CH₃CO⁺ |
| **5** | Spectre RMN ¹H (DMSO-d6, δ en ppm) : 2,16 (s, 3H) ; 5,24 (s, 1H) ; 5,61 (s, 1H) ; 7,18 (t, J = 9,0 Hz, 2H) ; 7,61 (d, J = 9,5 Hz, 1H) ; 7,70 (dd, J = 2,0 et 9,5 Hz, 1H) ; 7,92 (dd, J = 5,5 et 9,0 Hz, 2H) ; 8,47 (s, 1H) ; 8,73 (s large, 1H) ; 10,35 (s, 1H). Spectre de masse (LC/MS) : m/z 296, [M+H]⁺ |
| **6** | Spectre RMN ¹R (DMSO-d6, δ en ppm) : 9,91 (s, 1H), 8,91 (s, 1H), 8,53 (s, 1H), 8,31 (d, J = 6,9, 1H), 7,77 (d, J = 9,6, 1H), 7,56 (d, J = 8,0, 1H), 7,49-7,39 (m, 2H), 7,23-7,18 (m, 1H). Spectre de masse (IC) : m/z 307 : [M+H]⁺ |
| **7** | Spectre RMN ¹H (DMSO-d6, δ en ppm) : 7,10 (t large, J = 7,5 Hz, 1H) ; 7,35 ( t large, J = 7,5 Hz, 2H) ; 7,44 (t large, J = 7,5 Hz, 1H) ; 7,54 ( t large, J = 7,5 Hz, 2H) ; de 7,71 à 7,77 (m, 4H) ; 7,91 (d large, J = 8,0 Hz, 2H) ; 8,52 (s, 1H) ; 9,00 (s large, 1H) ; 10,3 (s large, 1H). Spectre IR (KBr) : 3360 ; 1677 ; 1597 ; 11556 ; 1525 ; 1505 ; 1489 ; 1443 ; 1421 ; 1314 ; 1226 ; 762 & 693 cm⁻¹. Spectre de masse (IE) : m/z=313 [M]⁺, m/z=221 [M-NHPh]⁺ (pic de base). |
| **8** | Spectre RMN ¹H (DMSO-d6, δ en ppm) : 5,39 (d, J = 11,0 Hz, 1H) ; 5,92 (d, J = 17,5 Hz, 1H) ; 6,77 (dd, J = 11,0 et 17,5 Hz, 1H) ; 7,09 (t large, J = 7,5 Hz, 1H) ; 7,34 (t large, J = 7,5 Hz, 2H) ; 7,64 (d, J = 9,5 Hz, 1H) ; 7,70 (dd, J = 2,0 et 9,5 Hz, 1H) ; 7,89 (d large, J = 8,0 Hz, 2H) ; 8,47 (s, 1H) ; 8,66 (s large, 1H) ; 10,2 (s, 1H). Spectre de masse (IE) : m/z 263 [M]⁺. |
| **9** | Spectre RMN ¹R (DMSO-d6, δ en ppm) : 1,24 (t, J = 7,5 Hz, 3H) ; 2,64 (q, J =7,5 Hz, 2H) ; 7,08 (t, J = 8,0 Hz, 1H) ; de 7,29 à 7,36 (m, 3H) ; 7,59 (d, J = 9,0 Hz, 1H) ; 7,89 (d large, J = 8,0 Hz, 2H) ; 8,43 (s, 1H) ; 8,44 (s large, 1H) ; 10,2 (s, 1H). |
| **10** | Spectre RMN ¹H (DMSO-d6, δ en ppm) : 7,11 (t, J = 8,0 Hz, 1H) ; 7,36 (t large, J = 8,0 Hz, 2H) ; 7,71 (dd, J = 1,5 et 9,5 Hz, 1H) ; 7,77 (d large, J = 9,5 Hz, 1H) ; 7,90 (d large, J = 8,0 Hz, 2H) ; 8,73 (s, 1H) ; 9,39 (s large, 1H) ; 10,0 (s, 1H) ; 10,35 (s large, 1H). Spectre de masse (LC/MS) : m/z 266,[M+H⁺] |
| **11** | Spectre RMN ¹R (DMSO-d6, δ en ppm) : 4,37 (s, 1H) ; 7,09 (t, J = 8,0 Hz, 1H) ; 7,34 (t large, J = 8,0 Hz, 2H) ; 7,39 (d large, J = 9,5 Hz, 1H) ; 7,67 (d, J = 9,5 Hz, 1H) ; 7,89 (d large, J = 8,0 Hz, 2H) ; 8,48 (s, 1H) ; 8,92 (s large, 1H) ; 10,3 (s, 1H). Spectre de masse (IE) : m/z 261 [M]⁺ (pic de base), m/z=221 [M-NHPh]⁺. |
| **12** | Spectre RMN ¹R (DMSO-d6, δ en ppm) : 1,50 (s, 6H) ; 5,10 (s, 1H) ; 7,10 (t, J = 8,0 Hz, 1H) ; 7,35 (t large, J = 8,0 Hz, 2H) ; 7,45 (t, J = 8,0 Hz, 1H) ; 7,54 (m, 2H) ; 7,75 (m, 2H); 7,82 (s large, 1H) ; 7,91 (d large, J = 8,0 Hz, 2H) ; 8,54 (s, 1H) ; 8,98 (s large, 1H) ; 10,3 (s, 1H). Spectre de masse (ES) : m/z 372, [M+H]⁺ (pic de base) |
| **13** | Spectre RMN ¹H (DMSO-d6, δ en ppm) : 5,78 (s, 1H) ; 6,20 (s, 1H) ; 7,08 (t, J = 8,0 Hz, 1H) ; 7,22 (d large, J = 9,5 Hz, 1H) ; 7,26 (t large, J = 8,0 Hz, 1H) ; de 7,30 à 7,38 (m, 4H) ; 7,44 (d large, J = 8,0 Hz, 2H) ; 7,56 (d, J = 9,5 Hz, 1H) ; 7,89 (d large, J = 8,0 Hz, 2H) ; 8,56 (s, 1H) ; 8,70 (s large, 1H) ; 10,2 (s, 1H). Spectre de masse (LC/MS) : m/z 344, [M+H]⁺ |
| **14** | Spectre RMN ¹H (DMSO-d6, δ en ppm) : 2,63 (s, 3H) ; 7,11 (t, J = 7,5 Hz, 1H) ; 7,36 (t large, J = 7,5 Hz, 2H) ; 7,73 (d, J = 10,0 Hz, 1H) ; 7,85 (dd, J = 2,0 et 10,0 Hz, 1H) ; 7,89 (d large, J = 7,5 Hz, 2H) ; 8,67 (s, 1H) ; 9,56 (s large, 1H) ; 10,4 (s, 1H). Spectre de masse (ES) : m/z 280, [M+H]⁺ (pic de base) |
| **15** | Spectre RMN 1H (DMSO-d6, δ en ppm) : 1,27 (d, J = 7,0 Hz, 6H) ; 2,95 (m, 1H) ; 7,09 (t, J = 7,5 Hz, 1H) ; 7,33 (t, J = 7,5 Hz, 2H) ; 7,38 (dd, J = 1,5 et 9,5 Hz, 1H) ; 7,60 (d, J = 9,5 Hz, 1H) ; 7,90 (d, J = 7,5 Hz, 2H) ; 8,43 (s, 1H) ; 8,47 (s large, 1H) ; 10,2 (s, 1H). Spectre de masse (LC-MS-DAD-ELSD) : m/z 280 [M+H]⁺ |
| **16** | Spectre RMN 1H (DMSO-d6, δ en ppm) : 1,40 (d, J = 6,5 Hz, 3H) ; 4,79 (m, 1H) ; 5,42 (d, J = 4,0 Hz, 1H) ; 7,09 (t, J = 8,0 Hz, 1H) ; 7,33 (t, J = 8,0 Hz, 2H) ; 7,39 (d large, J = 9,5 Hz, 1H) ; 7,61 (d, J = 9,5 Hz, 1H) ; 7,89 (d, J = 8,0 Hz, 2H) ; 8,51 (s, 1H) ; 8,56 (s large, 1H) ; 10,2 (s, 1H). Spectre de masse (LC-MS-DAD-ELSD) : m/z 282 [M+H]⁺ |
| **17** | Spectre RMN ¹H (DMSO-d6, δ en ppm) : 2,10 (s, 3H) ; 7,09 (t, J = 7,5 Hz, 1H) ; 7,30 (dd, J = 1,5 et 9,5 Hz, 1H) ; 7,33 (t, J = 7,5 Hz, 2H) ; 7,63 (d, J = 9,5 Hz, 1H) ; 7,88 (d, J = 7,5 Hz, 2H) ; 8,58 (s, 1H) ; 9,29 (s large, 1H) ; 10,15 (s, 1H). Spectre de masse (LC-MS-DAD-ELSD) : m/z 295 [M+H]⁺ |
| **18** | Spectre RMN 1H (DMSO-d6, δ en ppm) : 2,65 (s, 3H) ; 2,67 (s, 6H) ; 7,09 (t, J = 7,5 Hz, 1H) ; 7,34 (t, J = 7,5 Hz, 2H) ; 7,52 (d, J = 9,5 Hz, 1H) ; 7,58 (d, J = 9,5 Hz, 1H) ; 7,90 (d, J = 7,5 Hz, 2H) ; 8,34 (s, 1H) ; 10,2 (s, 1H). Spectre de masse (LC-MS-DAD-ELSD) : m/z 295 [M+H]⁺, m/z 220 [MH-Ph]⁺, m/z 202 [MH-NHPh]⁺. |
| **19** | Spectre RMN ¹R (DMSO-d6, δ en ppm) : 2,30 (s, 3H) ; 7,08 (t large, J = 7,5 Hz, 1H) ; 7,25 (dd, J = 2,0 et 9,5 Hz, 1H) ; 7,34 (t large, J = 7,5 Hz, 2H) ; 7,57 (d, J = 9,5 Hz, 1H) ; 7,89 (d large, J = 8,0 Hz, 2H) ; 8,42 (s large, 2H) ; 10,2 (s large, 1H) Spectre de masse (IE) : m/z 251[M⁺], m/z 159 (pic de base) : [M-NHPh]⁺, m/z 223 : [M-CO]⁺. |
| **20** | Spectre RMN ¹H (DMSO-d6, δ en ppm) : 2,68 (s, 3H) ; 6,90 (d large, J = 7,0 Hz, 1H) ; 7,09 (t large, J = 7,5 Hz, 1H) ; de 7,30 à 7,40 (m, 3H) ; 7,57 (d large, J = 9,0 Hz, 1H) ; 7,91 (d large, J = 8,0 Hz, 2H) ; 8,41 (s large, 1H) ; 10,3 (s large, 1H) Spectre de masse (IE) : m/z 251 (pic de base) : [M⁺], m/z 159 : [M+]-NHPh. |
| **21** | Spectre RMN ¹H (DMSO-d6, δ en ppm) : 2,39 (s, 3H) ; 6,87 (dd, J = 1,5 et 7,0 Hz, 1H) ; 7,08 (t large, J = 7,5 Hz, 1H) ; 7,33 (t large, J = 7,5 Hz, 2H); 7,41 (s large, 1H) ; 7,88 (d large, J = 8,0 Hz, 2H) ; 8,42 (s, 1H) ; 8,49 (d large, J = 7,0 Hz, 1H) ; 10,15 (s large, 1H). Spectre de masse (IE) : m/z 251 (pic de base) : [M]⁺, m/z 159 : [M-NIPh]⁺, m/z 223 : [M-CO]⁺. |
| **22** | Spectre RMN ¹H (DMSO-d6, δ en ppm) : 7,10 (t large, J = 7,5 Hz, 1H) ; 7,35 (t large, J = 7,5 Hz, 2H) ; 7,51 (dd, J = 2,0 et 9,5 Hz, 1H) ; 7,65 (d, J = 9,5 Hz, 1H) ; 7,88 (d large, J = 8,0 Hz, 2H) ; 8,47 (s, 1H) ; 8,99 (d, J = 2,0 Hz, 1H) ; 10,3 (s large, 1H) Spectre de masse (IE) : m/z 315 (pic de base) : [M]⁺, m/z 223 : [M-NHPh]⁺, m/z 287 : [M-CO]⁺. |
| **23** | Spectre RMN ¹H (DMSO-d6, δ en ppm) : 7,10 (t large, J = 7,5 Hz, 1H) ; 7,34 (t large, J = 7,5 Hz, 2H) ; 7,50 ddd, J = 2,5 - 8,0 et 10,0 Hz, 1H) ; 7,73 (dd, J = 5,0 et 10,0 Hz, 1H) ; 7,89 (d large, J = 8,5 Hz, 2H) ; 8,50 (s large, 1H) ; 8,83 (dd large, J = 2,5 et 4,5 Hz, 1H) ; 10,25 (s large, 1H). Spectre de masse (IE) : m/z 255 (pic de base) : [M]⁺, m/z 163 : [M-NHPh]⁺, m/z 227 : [M-CO]⁺. Spectre IR (KBr) : 3384; 3136 ; 1674; 1559 ; 1504 ; 1222 ; 1167 ; 797; 756, 687 cm⁻¹. |
| **24** | Spectre RMN ¹H (DMSO-d6, δ en ppm) : 7,10 (t large, J = 7,5 Hz, 1H) ; 7,35 (t large, J = 7,5 Hz, 2H) ; 7,69 ( ddd, J = 2,0 - 9,0 et 11,5 Hz, 1H) ; 7,88 (d large, J = 8,0 Hz, 2H) ; 8,63 (d, J = 3,0 Hz, 1H) ; 8,77 (m, 1H) ; 10,3 (s large, 1H) Spectre de masse (IE) : m/z 273 : [M]⁺, m/z 181 (pic de base) : [M-NHPh]⁺, m/z 245 : [M-CO]⁺. Spectre IR (KBr) : 3366 ; 3149 ; 1676 ; 1596 ; 1559 ; 1503; 1437 ; 1335 ; 1224 ; 1142 ; 1109 ; 883; 851& 767 cm⁻¹. |
| **25** | Spectre RMN ¹H (DMSO-d6, δ en ppm) : 2,81 (s, 3H) ; 7,10 (t large, J = 7,5 Hz, 1H) ; 7,35 (t large, J = 7,5 Hz, 2H) ; 7,53 ( d large, J = 9,5 Hz, 1H) ; 7,58 (d, J = 9,5 Hz, 1H) ; 7,89 (d large, J = 8,0 Hz, 2H) ; 8,53 (s, 1H) ; 10,3 (s large, 1H) Spectre de masse (IE) : m/z 329 (pic de base) : [M⁺], m/z 237 : [M-NHPh]⁺, m/z 301 : [M-CO]⁺. |
| **26** | Spectre RMN ¹H (DMSO-d6, δ en ppm) : 7,09 (t, J = 7,5 Hz, 1H) ; 7,34 (t, J = 7,5 Hz, 2H) ; 7,51 (d, J = 9,5 Hz, 1H) ; 7,57 (dd, J = 1,5 et 9,5 Hz, 1H) ; 7,88 (d, J = 8,0 Hz, 2H) ; 8,42 (s, 1H) ; 9,02 (s large, 1H) ; 10,25 (s, 1H). Spectre IR (KBr) : 3380 ; 1674; 1596 ; 1557 ; 1533 ; 1442 ; 1314& 790 cm⁻¹ Spectre de masse (IE): m/z=363 [M]⁺ (pic de base), m/z=271 [M- C₆H₆N]⁺, m/z=144 [m/z=271 - I]⁺. |
| **27** | Spectre RMN ¹H (DMSO-d6, δ en ppm) : 7,11 (t, J = 7,5 Hz, 1H) ; 7,35 (t, J = 7,5 Hz, 2H) ; 7,65 (dd, J = 2,0 et 9,5 Hz, 1H) ; 7,81 (d, J = 9,5 Hz, 1H) ; 7,89 (d, J = 8,0 Hz, 2H) ; 8,58 (s, 1H) ; 9,41 (s large, 1H) ; 10,4 (s large, 1H) Spectre IR (KBr) : 3364 ; 2234 ; 1671 ; 1599 ; 1560 ; 1527 ; 1504 ; 1433 & 748 cm⁻¹ Spectre de masse (IE): m/z=262 [M]⁺ (pic de base), m/z=170 [M-NHPh]⁺, m/z=143 [m/z-=170 - HCN]⁺. |
| **28** | Spectre RMN ¹H (DMSO-d6, δ en ppm) : 4,54 (d, J = 5,5 Hz, 2H) ; 5,43 (t, J = 5,5 Hz, 1H) ; 7,09 (t, J = 8,0 Hz, 1H) ; 7,34 (m, 3H) ; 7,62 (d, J = 9,5 Hz, 1H) ; 7,89 (d large, J = 8,0 Hz, 2H) ; 8,53 (s, 1H) ; 8,54 (s large, 1H) ; 10,2 (s, 1H). |
| **29** | Spectre RMN ¹H (DMSO-d6, δ en ppm) : 3,82 (s, 3H) ; 7,08 (t large, J = 7,5 Hz, 1H) ; 7,19 (dd, J = 2,5 et 9,5 Hz, 1H) ; 7,34 (t large, J = 7,5 Hz, 2H) ; 7,58 (d, J = 9,5 Hz, 1H) ; 7,88 (d large, J = 8,0 Hz, 2H) ; 8,32 (d, J = 2,5 Hz, 1H) ; 8,41 (s, 1H) ; 10,15 (s, 1H). Spectre de masse (ES) : m/z 268 [M+H]⁺. Spectre IR (KBr) 3344; 3140 ; 2841 ; 1664; 1594 ; 1552 ; 1539 ; 1505 ; 1446 ; 1319 ; 1215 ; 1022 ; 986 ; 801 ; 762 & 692 cm⁻¹ |
| **30** | Spectre RMN ¹H (DMSO-d6, δ en ppm) : 1,49 (s, 6H) ; 5,32 (s, 1H) ; 7,17 (t, J = 9,0 Hz, 1H) ; 7,49 (dd, J = 2,0 et 9,0 Hz, 2H) ; 7,58 (d, J = 9,0 Hz, 1H) ; 7,92 (dd, J = 5,0 et 9,0 Hz, 2H) ; 8,52 (s, 1H) ; 8,62 (s large, 1H) ; 10,35 (s, 1H). Spectre de masse (ES) : m/z = 203 [M - C₆H₅FN]⁺ (pic de base), m/z=313 [M]⁺ |
| **31** | Spectre RMN ¹H (DMSO-d6, δ en ppm) : 7,10 (t, J = 8,0 Hz, 1H) ; 7,35 (t large, J = 8,0 Hz, 2H) ; 7,62 (t, J = 8,0 Hz, 2H) ; de 7,71 à 7,82 (m, 3H) ; 7,88 (d large, J = 8,0 Hz, 2H) ; 7,91 (d large, J = 8,0 Hz, 2H) ; 8,65 (s, 1H) ; 9,17 (s large, 1H) ; 10,35 (s, 1H). |
| **32** | Spectre RMN ¹H (DMSO-d6, δ en ppm) : 2,16 (s, 3H) ; 5,24 (s large, 1H) ; 5,62 (s, 1H) ; 7,09 (t large, J = 7,5 Hz, 1H) ; 7,34 ( t large, J = 7,5 Hz, 2H) ; 7,61 (d large, J = 9,5 Hz, 1H) ; 7,70 (dd, J = 2,0 et 9,5 Hz, 1H) ; 7,88 (d large, J = 8,0 Hz, 2H) ; 8,48 (s, 1H) ; 8,73 (s large, 1H) ; 10,2 (s large, 1H). Spectre IR (KBr) : 3331 ; 1673 ; 1594 ; 1533 ; 1524 ; 1504 ; 1443 ; 1428 ; 1314 ; 1207 ; 756 & 692 cin7'. Spectre de masse (IE) : m/z=277 [M]+, m/z=185 [M-NHPh]⁺ (pic de base). |
| **33** | Spectre RMN ¹H (DMSO-d6, δ en ppm) : 6,92 (dt, J = 2,0 et 8,5 Hz, 1H) ; 7,37 (m, 1H) ; 7,45 (dd, J = 2,0 et 10,0 Hz, 1H) ; de 7,68 à 7,78 (m, 2H) ; 7,86 (td, J = 2,0 et 11,5 Hz, 1H) ; 8,50 (s, 1H) ; 8,92 (s large, 1H); 10,6 (s, 1H). Spectre de masse (IC) : m/z 289, [M⁺] |
| **34** | Spectre RMN ¹H (DMSO-d6, δ en ppm) : 7,15 (d large, J = 8,0 Hz, 1H) ; 7,37 (t, J = 8,0 Hz, 1H) ; 7,46 (dd, J = 2,0 et 9,5 Hz, 1H) ; 7,71 (d, J = 9,5 Hz, 1H) ; 7,85 (d large, J = 8,0 Hz, 1H) ; 8,10 (s large, 1H) ; 8,50 (s, 1H) ; 8,91 (s large, 1H) ; 10,6 (s, 1H). Spectre de masse (IC) : m/z 305, [M]⁺ |
| **35** | Spectre RMN ¹H (DMSO-d6, δ en ppm) : 3,76 (s, 3H) ; 6,68 (dd, J = 2,5 et 8,5 Hz, 1H) ; 7,24 (t, J = 8,5 Hz, 1H) ; 7,45 (dd, J = 2,0 et 9,5 Hz, 1H) ; 7,49 (d large, J = 8,5 Hz, 1H) ; 7,59 (t, J = 2,5 Hz, 1H) ; 7,70 (d, J = 9,5 Hz, 1H) ; 8,47 (s, 1H) ; 8,91 (s large, 1H) ; 10,25 (s, 1H). Spectre de masse (IC) : m/z 301, [M]⁺ |
| **36** | Spectre RMN ¹H (DMSO-d6, δ en ppm) : 2,87 (s, 6H) ; 6,72 (d, J = 9,0 Hz, 2H) ; 7,43 (dd, J = 2,0 et 9,5 Hz, 1H) ; 7,67 (d, J = 9,0 Hz, 2H) ; 7,69 (d, J = 9,5 Hz, 1H) ; 8,42 (s, 1H) ; 8,91 (s large, 1H) ; 10,0 (s, 1H). Spectre de masse (IC) : m/z 315, [M+H]⁺ |
| **37** | Spectre RMN ¹H (DMSO-d6, δ en ppm) : 7,40 (d, J = 9,0 Hz, 2H) ; 7,45 (d large, J = 10,0 Hz, 1H) ; 7,71 (d, J = 10,0 Hz, 1H) ; 7,95 (d, J = 9,0 Hz, 2H) ; 8,49 (s, 1H) ; 8,91 (s large, 1H) ; 10,5 (s, 1H). Spectre de masse (IC) : m/z 305, [M]⁺ |
| **38** | Spectre RMN ¹H (DMSO-d6, δ en ppm) : 4,47 (d, J = 5,5 Hz, 2H) ; 5,23 (t, J = 5,5 Hz, 1H) ; 7,10 (t, J = 7,5 Hz, 1H) ; de 7,31 à 7,50 (m, 6H) ; 7,60 (d, J = 8,0 Hz, 1H) ; 7,70 (d, J = 9,5 Hz, 1H) ; 7,92 (d, J = 8,0 Hz, 2H) ; 8,53 (s, 1H) ; 8,66 (s, 1H) ; 10,3 (s large, 1H). Spectre IR (KBr) : 3374 ; 1668 ; 1602 ; 1560 ; 1526 ; 1502 ; 1444 ; 1420 ; 1317 ; 754 & 691 cm⁻¹ Spectre de masse (ES) : m/z=344 [M+H]⁺ (pic de base). |
| **39** | Spectre RMN ¹H (DMSO-d6, δ en ppm) : 4,61 (d, J = 5,5 Hz, 2H) ; 5,29 (t large, J = 5,5 Hz, 1H) ; 7,10 (t large, J = 7,5 Hz, 1H) ; de 7,31 à 7,41 (m, 3H) ; 7,48 (t, J = 8,0 Hz, 1H) ; 7,60 (d large, J = 8,0 Hz, 1H) ; 7,68 (s large, 1H) ; 7,74 (s large, 2H) ; 7,91 (d large, J = 8,0 Hz, 2H) ; 8,54 (s, 1H) ; 8,99 (s large, 1H) ; 10,25 (s large, 1H). Spectre IR (KBr) : 3373 ; 1667; 1602 ; 1560 ; 1535 ; 1503 ; 1443 ; 1413 ; 1320 ; 1208 & 755 cm⁻¹. Spectre de masse (ES) : m/z=344 [M+H]⁺ (pic de base). |
| **40** | Spectre RMN ¹H (DMSO-d6, δ en ppm) : 4,57 (d, J = 5,5 Hz, 2H) ; 5,25 (t large, J = 5,5 Hz, 1H) ; 7,10 (t large, J = 7,5 Hz, 1H) ; 7,35 (t large, J = 7,5 Hz, 2H) ; 7,47 (d, J = 8,5 Hz, 2H) ; 7,70 (d, J = 8,5 Hz, 2H) ; 7,74 (s large, 2H) ; 7,91 (d large, J = 8,5 Hz, 2H) ; 8,51 (s, 1H) ; 8,99 (s large, 1H) ; 10,25 (s large, 1H). Spectre IR (KBr) : 3353 ; 1664; 1599; 1558 ; 1529 ; 1500 ; 1443 ; 1432; 1316; 1244 ; 802 & 755 cm⁻¹. Spectre de masse (ES) : m/z=344 [M+H]⁺ (pic de base). |
| **41** | Spectre RMN ¹H (DMSO-d6, δ en ppm) : 7,10 (t large, J = 7,5 Hz, 1H) ; 7,35 (t large, J = 7,5 Hz, 2H) ; 7,52 (dd, J = 2,0 et 9,5 Hz, 1H) ; 7,64 (d large, J = 7,5 Hz, 1H) ; 7,68 (t large, J = 7,5 Hz, 1H) ; 7,75 (d, J = 9,5 Hz, 1H) ; 7,82 (t large, J = 7,5 Hz, 1H) ; 7,92 (d large, J = 8,0 Hz, 2H) ; 8,01 (d large, J = 8,0 Hz, 1H) ; 8,54 (s, 1H) ; 8,78 (s large, 1H) ; 10,5 (s, 1H) ; 10,3 (s large, 1H). Spectre de masse (ES) : m/z 342 [M+H]⁺. |
| **42** | Spectre RMN ¹H (DMSO-d6, δ en ppm) : 7,10 (t large, J = 7,5 Hz, 1H) ; 7,35 (t large, J = 7,5 Hz, 2H) ; de 7,74 à 7,86 (m, 3H) ; 7,91 (d large, J = 8,0 Hz, 2H) ; 7,98 (d large, J = 7,5 Hz, 1H) ; 8,10 (d large, J = 8,0 Hz, 1H) ; 8,28 (s large, 1H) ; 8,54 (s, 1H) ; 9,13 (s large, 1H) ; 10,15 (s, 1H) ; 10,3 (s large, 1H). Spectre IR (KBr) : 3353 ; 3155 ; 2858 ; 2735 ; 1695 ; 1670 ; 1599 ; 1556 ; 1526 ; 1504 ; 1441 ; 1315 ; 1207 ; 797 ; 745 & 690 cm⁻¹. Spectre de masse (IE) : m/z 341 [M]⁺. |
| **43** | Spectre RMN ¹H (DMSO-d6, δ en ppm) : 2,34 (s, 3H) ; 2,61 (s, 3H) ; 7,09 (t large, J = 7,5 Hz, 1H) ; 7,29 (d, J = 9,5 Hz, 1H) ; 7,34 (t large, J = 7,5 Hz, 2H) ; 7,49 (d, J = 9,5 Hz, 1H) ; 7,90 (d large, J = 8,0 Hz, 2H) ; 8,37 (s, 1H) ; 10,2 (s, 1H). Spectre de masse (IE) : m/z 265 (pic de base) : [M⁺], m/z 173 : [M⁺]-NHPh, m/z 237 : [M⁺]-[CO]. |
| **44** | Spectre RMN ¹H (DMSO-d6, δ en ppm) : 3,92 (s, 3H) ; 7,10 (t, J = 8,0 Hz, 1H) ; 7,35 ( larget, J = 8,0 Hz, 2H) ; 7,70 (t, J = 7,5 Hz, 1H) ; de 7,75 à 7,82 (m, 2H) ; 7,91 (d large, J = 8,0 Hz, 2H) ; de 8,00 à 8,06 (m, 2H) ; 8,28 (t, J = 2,0 Hz, 1H) ; 8,54 ( s, 1H) ; 9,10 (s large, 1H) ; 10,3 (s, 1H). Spectre de masse (ES) : m/z=372 [M+H]⁺ (pic de base) |
| **45** | Spectre RMN ¹H (DMSO-d6, δ en ppm) : 2,68 (s, 3H) ; 7,10 (t, J = 7,5 Hz, 1H) ; 7,36 (t large, J = 7,5 Hz, 2H) ; 7,70 (t, J = 7,5 Hz, 1H) ; 7,77 (d, J = 9,5 Hz, 1H) ; 7,83 (dd, J = 2,0 et 9,5 Hz, 1H) ; 7,91 (d large, J = 7,5 Hz, 2H) ; 8,02 (d large, J = 7,5 Hz, 2H) ; 8,28 (t, J = 2,0 Hz, 1H) ; 8,54 (s, 1H) ; 9,11 (s large, 1H) ; 10,25 (s, 1H). Spectre de masse (ES) : m/z=356 [M+H]⁺ (pic de base) |
| **46** | Spectre RMN ¹H (DMSO-d6, δ en ppm) : 7,10 (t, J = 8,0 Hz, 1H) ; 7,27 (m, 1H) ; 7,35 (t large, J = 8,0 Hz, 2H) ; de 7,54 à 7,65 (m, 3H) ; 7,75 (d, J = 9,0 Hz, 1H) ; 7,78 (dd, J = 2,0 et 9,0 Hz, 1H) ; 7,91 (d large, J = 8,0 Hz, 2H) ; 8,50 (s, 1H) ; 9,07 (s large, 1H) ; 10,3 (s, 1H). Spectre de masse (IE) : m/z 239 : [M-C₆H₆N⁺] (pic de base), m/z 331 : [M⁺] |
| **47** | Spectre RMN ¹H (DMSO-d6, δ en ppm) : 2,37 (s, 3H) ; 7,10 (t, J = 8,0 Hz, 1H) ; de 7,31 à 7,38 (m, 4H) ; 7,63 (d, J = 8,0 Hz, 2H) ; 7,73 (d, J = 2,0 Hz, 1H) ; 7,91 (d large, J = 8,0 Hz, 2H) ; 8,50 (s, 1H) ; 8,96 (s large, 1H) ; 10,25 (s, 1H). Spectre de masse (IE) : m/z 235 : [M-NHPh]⁺ (pic de base), m/z 327 : [M]⁺ |
| **48** | Spectre RMN ¹H (DMSO-d6, δ en ppm) : 3,85 (s, 3H) ; 7,01 (dd, J = 3,0 et 8,5 Hz, 1H) ; 7,10 (t, J = 8,0 Hz, 1H) ; de 7,27 à 7,31 (m, 2H) ; 7,35 (t large, J = 8,0 Hz, 2H) ; 7,45 (t, J = 8,5 Hz, 1H) ; de 7,71 à 7,78 (m, 2H) ; 7,91 (d large, J = 8,0 Hz, 2H) ; 8,50 (s, 1H) ; 9,01 (s large, 1H) ; 10,25 (s, 1H). Spectre de masse (IE) : m/z 251 : [M-NHPh]⁺ (pic de base), m/z 343 : [M]⁺ |
| **49** | Spectre RMN ¹H (DMSO-d6, δ en ppm) : 2,13 (m très étalé, 2H) ; 3,81 (s, 3H) ; 7,10 (t large, J = 8,0 Hz, 1H) ; 7,35 (t large, J = 8,0 Hz, 2H) ; 7,39 (d large, J = 7,5 Hz, 1H) ; 7,46 (t, J = 7,5 Hz, 1H) ; 7,57 (d large, J = 7,5 Hz, 1H) ; 7,71 (s large, 1H) ; 7,75 (d, J = 1,5 Hz, 1H) ; 7,91 (d large, J = 8,0 Hz, 2H) ; 8,53 (s, 1H) ; 8,99 (s large, 1H) ; 10,3 (s, 1H). Spectre de masse (ES) : m/z 343, [M+H]⁺ (pic de base) |
| **50** | Spectre RMN ¹H (DMSO-d6, δ en ppm) : 7,10 (t, J = 8,0 Hz, 1H) ; 7,35 (t large, J = 8,0 Hz, 2H) ; 7,51 (d large, J = 8,0 Hz, 1H) ; 7,57 (t, J = 8,0 Hz, 1H) ; de 7,71 à 7,81 (m, 3H) ; 7,84 (s large, 1H) ; 7,91 (d large, J = 8,0 Hz, 2H) ; 8,50 (s, 1H) ; 9,08 (s large, 1H) ; 10,3 (s, 1H). Spectre de masse (ES) : m/z 348, [M+H]⁺ (pic de base) |
| **51** | Spectre RMN ¹H (DMSO-d6, δ en ppm) : 7,10 (t, J = 8,0 Hz, 1H) ; 7,35 (t, J = 8,0 Hz, 2H) ; 7,48 (m large, 1H) ; 7,61 (t, J = 8,0 Hz, 1H) ; 7,80 (m, 2H) ; 7,91 (m, 4H) ; 8,12 (m large, 1H) ; 8,24 (s large, 1H) ; 8,54 (s, 1H) ; 9,08 (s large, 1H) ; 10,3 (s, 1H). Spectre de masse (ES) : m/z 357, [M+H]⁺ (pic de base) |
| **52** | Spectre RMN ¹H (DMSO-d6, δ en ppm) : 1,39 (d, J = 6,5 Hz, 3H) ; 4,83 (m, 1H) ; 5,29 (d, J = 4,0 Hz, 1H) ; 7,09 (t, J = 8,0 Hz, 1H) ; 7,36 (t large, J = 8,0 Hz, 2H) ; 7,40 (d large, J = 8,0 Hz, 1H) ; 7,48 (t, J = 8,0 Hz, 1H) ; 7,58 (d large, J = 8,0 Hz, 1H) ; 7,70 (s large, 1H) ; 7,76 (m, 2H) ; 7,90 (d large, J = 8,0 Hz, 2H) ; 8,54 (s, 1H) ; 8,99 (s large, 1H) ; 10,3 (s, 1H). Spectre de masse (ES) : m/z 358, [M+H⁺] (pic de base) |
| **53** | Spectre RMN 1H (DMSO-d6, δ en ppm) : 2,41 (s, 3H) ; 7,10 (t, J = 7,5 Hz, 1H) ; 7,26 (d large, J = 8,0 Hz, 1H) ; 7,35 (t, J = 7,5 Hz, 2H) ; 7,42 (t, J = 8,0 Hz, 1H) ; 7,53 (d large, J = 8,0 Hz, 1H) ; 7,57 (s large, 1H) ; 7,74 (s, 2H) ; 7,91 (d, J = 7,5 Hz, 2H) ; 8,51 (s, 1H) ; 8,99 (s, 1H) ; 10,3 (s, 1H). Spectre de masse (LC-MS-DAD-ELSD) : m/z 328 [M+H]⁺ |
| **54** | Spectre RMN 1H (DMSO-d6, δ en ppm) : 1,12 (t, J = 7,0 Hz, 6H) ; de 3,25 à 3,45 (m masqué, 4H) ; 7,14 (t, J = 8,0 Hz, 1H) ; 7,39 (t, J = 8,0 Hz, 2H) ; 7,62 (s large, 2H) ; 7,82 (d, J = 8,0 Hz, 2H) ; 8,14 (m étalé, 1H) ; 8,61 (s large, 1H) ; 10,65 (m étalé, 1H). Spectre de masse (LC-MS-DAD-ELSD) : m/z 309 [M+H]⁺ |
| **55** | Spectre RMN 1H (DMSO-d6, δ en ppm) : 2,83 (d, J = 6,5 Hz, 3H) ; 7,11 (t, J = 8,0 Hz, 1H) ; 7,37 (t, J = 8,0 Hz, 2H) ; 7,62 (t, J = 8,0 Hz, 1H) ; 7,80 (d large, J = 8,0 Hz, 1H) ; de 7,33 à 7,95 (m, 5H) ; 8,20 (s large, 1H) ; 8,59 (m, 2H) ; 9,11 (s, 1H) ; 10,35 (s, 1H). Spectre de masse (LC-MS-DAD-ELSD) : m/z 371 [M+H]⁺, m/z 415 [M+HCO₂]⁻ |
| **56** | Spectre RMN 1H (DMSO-d6, δ en ppm) : 7,10 (t, J = 7,5 Hz, 1H) ; 7,34 (t, J = 7,5 Hz, 2H) ; 7,60 (m étalé, 1H) ; 7,69 (d, J = 9,5 Hz, 1H) ; 7,80 (dd, J = 1,5 et 9,5 Hz, 1H) ; 7,89 (d, J = 7,5 Hz, 2H) ; 8,14 (m étalé, 1H) ; 8,61 (s, 1H) ; 9,20 (s large, 1H); 10,3 (s, 1H). |
| **57** | Spectre RMN 1H (DMSO-d6, δ en ppm) : 2,87 (s, 6H) ; 6,89 (dt, J = 2,5 et 9,0 Hz, 1H) ; 7,35 (m, 2H) ; 7,51 (d, J = 10,0 Hz, 1H) ; 7,72 (dd large, J = 2,5 et 9,0 Hz, 1H) ; 7,88 (td, J = 2,5 et 9,0 Hz, 1H) ; 7,90 (s large, 1H) ; 8,34 (s, 1H) ; 13,35 (s large, 1H). Spectre de masse (LC-MS-DAD-ELSD) : m/z 299 [M+H]⁺. |
| **58** | Spectre RMN 1H (DMSO-d6, δ en ppm) : 2,88 (s, 6H) ; 7,00 (m, 1H) ; 7,39 (m, 2H) ; 7,55 (d, J = 9,5 Hz, 1H) ; 7,89 (d, J = 2,5 Hz, 1H) ; 8,07 (ddd, J = 3,5 - 6,5 et 10,5 Hz, 1H) ; 8,39 (s, 1H) ; 9,72 (s large, 1H). Spectre de masse (LC-MS-DAD-ELSD) : m/z 317 [M+H]⁺. |
| **59** | Spectre RMN 1H (DMSO-d6, δ en ppm) : 2,87 (s, 6H) ; 7,22 (m, 2H) ; 7,38 (dd, J = 2,5 et 10,0 Hz, 1H) ; 7,54 (d, J = 10,0 Hz, 1H) ; 7,83 (m, 1H) ; 7,89 (d, J = 2,5 Hz, 1H) ; 8,38 (s, 1H) ; 9,89 (s large, 1H). Spectre de masse (LC-MS-DAD-ELSD) : m/z 317 [M+H]⁺. |
| **60** | Spectre RMN 1H (DMSO-d6, δ en ppm) : 2,87 (s, 6H) ; de 7,12 à 7,36 (m, 3H) ; 7,39 (dd, J = 2,5 et 10,0 Hz, 1H) ; 7,56 (d, J = 10,0 Hz, 1H) ; 7,89 (d, J = 2,5 Hz, 1H) ; 8,15 (dt, J = 2,5 et 8,0 Hz, 1H) ; 8,37 (s, 1H) ; 9,69 (s large, 1H). Spectre de masse (LC-MS-DAD-ELSD) : m/z 299 [M+H]⁺. |
| **61** | Spectre RMN 1H (DMSO-d6, δ en ppm) : 4,59 (s large, 2H) ; 5,30 (s large, 1H) ; 6,92 (dt, J = 2,5 et 9,0 Hz, 1H) ; 7,38 (m, 2H) ; 7,49 (t, J = 7,5 Hz, 1H) ; 7,60 (d large, J = 7,5 Hz, 1H) ; 7,69 (s large, 1H) ; 7,76 (m, 3H) ; 7,89 (td, J = 2,5 et 11,5 Hz, 1H) ; 8,57 (s, 1H) ; 9,00 (s large, 1H) ; 10,55 (s large, 1H). Spectre de masse (LC-MS-DAD-ELSD) : m/z 362 [M+H]⁺. |
| **62** | Spectre RMN 1H (DMSO-d6, δ en ppm) : 4,60 (d, J = 5,5 Hz, 2H) ; 5,29 (t, J = 5,5 Hz, 1H) ; 6,93 (tt, J = 2,5 et 9,0 Hz, 1H) ; 7,39 (d large, J = 7,5 Hz, 1H) ; 7,49 (t, J = 7,5 Hz, 1H) ; 7,60 (d large, J = 7,5 Hz, 1H) ; 7,69. (s large, 1H) ; 7,75 (m, 4H) ; 8,59 (s, 1H) ; 9,00 (t, J = 1,5 Hz, 1H) ; 10,8 (s, 1H). Spectre de masse (LC-MS-DAD-ELSD) : m/z 380 [M+H]⁺. |
| **63** | Spectre RMN 1H (DMSO-d6, δ en ppm) : 4,60 (d, J = 5,5 Hz, 2H) ; 5,29 (t, J = 5,5 Hz, 1H) ; 7,21 (dt, J = 1,5 et 7,5 Hz, 1H) ; de 7,35 à 7,45 (m, 2H) ; 7,49 (t, J = 7,5 Hz, 1H) ; 7,59 (m, 2H) ; 7,69 (s large, 1H) ; de 7,72 à 7,85 (m, 2H) ; 8,38 (dd, J = 1,5 et 7,5 Hz, 1H) ; 8,60 (s, 1H) ; 8,99 (s large, 1H) ; 9,95 (s, 1H). Spectre de masse (LC-MS-DAD-ELSD) : m/z 378 [M+H]⁺, présence de 1 Cl. |
| **64** | Spectre RMN 1H (DMSO-d6, δ en ppm) : 2,31 (s, 3H) ; 4,60 (d, J = 5,5 Hz, 2H) ; 5,29 (t, J = 5,5 Hz, 1H) ; 6,92 (d large, J = 7,5 Hz, 1H) ; 7,22 (t, J = 7,5 Hz, 1H) ; 7,39 (d large, J = 7,5 Hz, 1H) ; 7,49 (t, J = 7,5 Hz, 1H) ; 7,60 (d large, J = 7,5 Hz, 1H) ; 7,68 (m, 2H) ; 7,73 (s, 2H) ; 7,76 (s large, 1H) ; 8,51 (s, 1H); 8,99 (t, J = 1,5 Hz, 1H) ; 10,15 (s, 1H). Spectre de masse (LC-MS-DAD-ELSD) : m/z 358 [M+H]⁺. |
| **65** | Spectre RMN 1H (DMSO-d6, δ en ppm) : 4,60 (d, J = 5,5 Hz, 2H) ; 5,29 (t, J = 5,5 Hz, 1H) ; 7,05 (m, 1H) ; 7,40 (m, 2H) ; 7,49 (t, J = 7,5 Hz, 1H) ; 7,60 (d large, J = 7,5 Hz, 1H) ; 7,69 (s large, 1H) ; de 7,72 à 7,84 (m, 2H) ; 8,02 (ddd, J = 2,5 - 6,0 Hz et 10,5 Hz, 1H) ; 8,59 (s, 1H) ; 8,99 (t, J = 1,5 Hz, 1H) ; 9,89 (s large, 1H). Spectre de masse (LC-MS-DAD-ELSD) : m/z 380 [M+H]⁺. |
| **66** | Spectre RMN 1H (DMSO-d6, δ en ppm) : 4,60 (d, J = 5,5 Hz, 2H) ; 5,29 (t, J = 5,5 Hz, 1H) ; 7,25 (m, 2H) ; 7,39 (d large, J = 7,5 Hz, 1H) ; 7,49 (t, J = 7,5 Hz, 1H) ; 7,60 (d large, J = 7,5 Hz, 1H) ; 7,69 (s large, 1H) ; de 7,72 à 7,83 (m, 3H) ; 8,58 (s, 1H) ; 8,99 (s large, 1H) ; 10,1 (s large, 1H). Spectre de masse (LC-MS-DAD-ELSD) : m/z 380 [M+H]⁺. |
| **67** | Spectre RMN 1H (DMSO-d6, δ en ppm) : 4,60 (d, J = 5,5 Hz, 2H) ; 5,29 (t, J = 5,5 Hz, 1H) ; de 7,18 à 7,37 (m, 3H) ; 7,39 (d large, J = 7,5 Hz, 1H); 7,49 (t, J = 7,5 Hz, 1H) ; 7,60 (d large, J = 7,5 Hz, 1H) ; 7,69 (s large, 1H) ; de 7,72 à 7,82 (m, 2H) ; 8,10 (m, 1H) ; 8,57 (s, 1H) ; 8,99 (t, J = 1,5 Hz, 1H) ; 9,83 (d, J = 1,5 Hz, 1H). Spectre de masse (LC-MS-DAD-ELSD) : m/z 362 [M+H]⁺. |
| **68** | Spectre RMN 1H (DMSO-d6, δ en ppm) : 4,60 (d, J = 5,5 Hz, 2H) ; 5,29 (t, J = 5,5 Hz, 1H) ; 7,28 (ddd, J = 2,0 - 4,5 et 8,5 Hz, 1H) ; 7,40 (m, 2H) ; 7,49 (t, J = 7,5 Hz, 1H) ; 7,60 (d large, J = 7,5 Hz, 1H) ; 7,69 (s large, 1H) ; 7,79 (m, 2H) ; 8,19 (dd, J = 2,0 et 6,5 Hz, 1H) ; 8,59 (s, 1H) ; 8,99 (s large, 1H) ; 9,91 (d, J = 1,5 Hz, 1H). Spectre de masse (LC-MS-DAD-ELSD) : m/z 396 [M+H]⁺, présence de 1 Cl. |
| **69** | Spectre RMN ¹H (DMSO-d6, δ en ppm) : 3,12 (m, 4H) ; 3,79 (m, 4H) ; 7,12 (t, J = 8,0 Hz, 1H) ; 7,38 (t, J = 8,0 Hz, 2H) ; de 7,60 à 7,74 (m, 2H) ; 7,83 (d, J = 8,0 Hz, 2H) ; 8,25 (s, 1H) ; 8,59 (s, 1H) ; 10,5 (m étalé, 1H). Spectre de masse (LC-MS-DAD-ELSD) : m/z 323 [M+H]⁺. |
| **70** | Spectre RMN 1H (DMSO-d6, δ en ppm) : 2,32 (m, 2H) ; 3,82 (t, J = 7,5 Hz, 4H) ; 6,91 (dd, J = 1,5 et 9,5 Hz, 1H) ; 7,08 (t, J = 7,5 Hz, 1H) ; 7,32 (t, J= 7,5 Hz, 2H) ; 7,52 (d, J = 9,5 Hz, 1H); 7,70 (d, J = 1,5 Hz, 1H) ; 7,89 (d, J = 7,5 Hz, 2H) ; 8,31 (s, 1H) ; 10,15 (s, 1H). Spectre de masse (LC-MS-DAD-ELSD) : m/z 293 [M+H]⁺. |
| **71** | Spectre RMN 1H (DMSO-d6, δ en ppm) : 2,89 (s, 3H) ; 7,10 (tt, J = 1,5 et 7,5 Hz, 1H) ; 7,33 (t, J = 7,5 Hz, 2H) ; 7,39 (d, J = 9,5 Hz, 1H) ; 7,70 (d, J = 9,5 Hz, 1H) ; 7,90 (d, J = 7,5 Hz, 2H) ; 8,49 (s, 1H) ; 10,3 (s, 1H). Spectre de masse (LC-MS-DAD-ELSD) : m/z 378 [M+H]⁺. |
| **72** | Spectre RMN 1H (DMSO-d6, δ en ppm) : 6,92 (tt, J = 2,0 et 9,0 Hz, 1H) ; 7,51 (d, J = 9,5 Hz, 1H) ; 7,59 (dd, J = 1,5 et 9,5 Hz, 1H) ; 7,72 (m, 2H) ; 8,47 (s, 1H) ; 9,02 (s large, 1H) ; 10,75 (s, 1H). Spectre de masse (LC-MS-DAD-ELSD) : m/z 398 [M+H]⁻; m/z 400 [M+H]⁺. |
| **73** | Spectre RMN 1H (DMSO-d6, δ en ppm) : 4,61 (s, 2H) ; 7,15 (dm, J = 8,0 Hz, 1H) ; 7,38 (m, 2H) ; 7,49 (t, J = 8,0 Hz, 1H) ; 7,61 (d large, J = 8,0 Hz, 1H) ; 7,69 (s large, 1H) ; 7,77 (m, 2H) ; 7,87 (dm, J = 8,0 Hz, 1H) ; 8,12 (t, J = 2,0 Hz, 1H) ; 8,58 (s, 1H) ; 9,01 (s large, 1H) ; 10,55 (s, 1H). Spectre de masse (UPLC-MS-DAD-ELSD) : m/z 376 [M+H]⁻; m/z 378 [M+H]⁺, présence de 1 Cl. |
| **74** | Spectre RMN 1H (DMSO-d6, δ en ppm) : 2,87 (s, 6H) ; 6,90 (tt, J = 2,5 et 9,5 Hz, 1H); 7,35 (dd, J = 2,5 et 10,0 Hz, 1H); 7,51 (d, J = 10,0 Hz, 1H); 7,72 (m, 2H) ; 7,89 (d, J = 2,5 Hz, 1H) ; 8,37 (s, 1H) ; 10,6 (s large, 1H). Spectre de masse (UPLC-MS-DAD-ELSD) : m/z 317 [M+H]⁺. |
| **75** | Spectre RMN 1H (DMSO-d6, δ en ppm) : 2,88 (s, 6H) ; 7,18 (dt, J = 1,5 et 7,5 Hz, 1H) ; de 7,32 à 7,43 (m, 2H) ; 7,57 (m, 2H) ; 7,89 (d, J = 2,5 Hz, 1H) ; 8,38 (s, 1H) ; 8,40 (dd, J = 1,5 et 7,5 Hz, 1H) ; 9,86 (s, 1H). Spectre de masse (UPLC-MS-DAD-ELSD) : m/z 315 [M+H]⁺, présence de 1 Cl. |
| **76** | Spectre RMN 1H (DMSO-d6, δ en ppm) : 2,87 (s, 6H) ; 7,04 (dm, J = 8,0 Hz, 1H) ; 7,37 (dd, J = 2,5 et 10,0 Hz, 1H) ; 7,44 (t, J = 8,0 Hz, 1H) ; 7,51 (d, J = 10,0 Hz, 1H) ; 7,89 (m, 2H) ; 8,09 (s large, 1H) ; 8,36 (s, 1H) ; 10,5 (s, 1H). Spectre de masse (UPLC-MS-DAD-ELSD) : m/z 365 [M+H]⁺. |
| **77** | Spectre RMN 1H (DMSO-d6, δ en ppm) : 4,61 (d, J = 6,0 Hz, 2H) ; 5,29 (t, J = 6,0 Hz, 1H) ; 7,27 (dt ; J = 1,5 et 8,0 Hz, 1H) ; de 7,35 à 7,44 (m, 2H) ; 7,49 (t, J = 8,0 Hz, 1H) ; 7,61 (d large, J = 8,0 Hz, 1H) ; 7,69 (s large, 1H) ; 7,78 (m, 2H) ; 7,94 (dt, J = 1,5 et 8,0 Hz, 1H) ; 8,58 (s, 1H) ; 8,99 (s large, 1H) ; 10,05 (s, 1H). Spectre de masse (UPLC-MS-DAD-ELSD) : m/z 394 [M+H]⁻; m/z 396 [M+H]⁺, présence de 1 Cl. |
| **78** | Spectre RMN 1H (DMSO-d6, δ en ppm) : 2,30 (s, 3H) ; 2,87 (s, 6H) ; 6,89 (d large, J = 7,5 Hz, 1H) ; 7,21 (t, J = 7,5 Hz, 1H) ; 7,33 (dd, J = 2,5 et 10,0 Hz, 1H) ; 7,50 (d, J = 10,0 Hz, 1H) ; 7,63 (d large, J = 7,5 Hz, 1H) ; 7,71 (s large, 1H) ; 7,89 (d, J = 2,5 Hz, 1H) ; 8,31 (s, 1H) ; 9,94 (s, 1H). Spectre de masse (UPLC-MS-DAD-ELSD) : m/z 295 [M+H]⁺ |
| **79** | Spectre RMN 1H (DMSO-d6, δ en ppm) : 2,87 (s, 6H) ; 7,12 (ddd, J = 1,0 - 2,0 et 8,0 Hz, 1H) ; 7,35 (m, 2H) ; 7,51 (d, J = 10,0 Hz, 1H) ; 7,83 (ddd, J = 1,0 - 2,0 et 8,0 Hz, 1H) ; 7,89 (d, J = 2,5 Hz, 1H) ; 8,11 (t, J = 2,0 Hz, 1H) ; 8,35 (s, 1H) ; 10,35 (s, 1H). Spectre de masse (UPLC-MS-DAD-ELSD) : m/z 315 [M+H]⁺, présence de 1 Cl. |
| **80** | Spectre RMN 1H (DMSO-d6, δ en ppm) : 2,88 (s, 6H); 7,24 (ddd, J = 2,5 - 4,5 et 9,0 Hz, 1H) ; 7,29 (m, 2H) ; 7,54 (d, J = 10,0 Hz, 1H) ; 7,89 (d, J = 2,5 Hz, 1H) ; 8,23 (dd, J = 2,5 et 6,5 Hz, 1H) ; 8,39 (s, 1H) ; 9,75 (d, J = 2,0 Hz, 1H). Spectre de masse (UPLC-MS-DAD-ELSD) : m/z 333 [M+H]⁺, présence de 1 Cl. |
| **81** | Spectre RMN 1H (DMSO-d6, δ en ppm) : 2,88 (s, 6H) ; 7,25 (dt, J = 1,5 et 8,0 Hz, 1H) ; 7,38 (m, 2H) ; 7,54 (d, J = 10,0 Hz, 1H) ; 7,89 (d, J = 2,5 Hz, 1H) ; 8,00 (dt, J = 1,5 et 8,0 Hz, 1H) ; 8,38 (s, 1H) ; 9,88 (s large, 1H). Spectre de masse (UPLC-MS-DAD-ELSD) : m/z 333 [M+H]⁺, présence de 1 Cl. |
| **82** | Spectre RMN 1H (DMSO-d6, δ en ppm) : 2,87 (s, 6H) ; 6,88 (dd, J = 2,5 et 8,0 Hz, 1H); 7,21 (t, J = 74,0 Hz, 1H) ; 7,38 (m, 2H) ; 7,51 (d, J = 10,0 Hz, 1H) ; 7,74 (dm, J = 8,0 Hz, 1H) ; 7,89 (m, 2H) ; 8,34 (s, 1H) ; 10,3 (s, 1H). Spectre de masse (UPLC-MS-DAD-ELSD) : m/z 347 [M+H]⁺. |
| **83** | Spectre RMN 1H (DMSO-d6, δ en ppm) : 2,88 (s, 6H) ; 7,36 (dd, J = 2,5 et 10,0 Hz, 1H) ; 7,41 (d large, J = 8,0 Hz, 1H) ; 7,52 (d, J = 10,0 Hz, 1H) ; 7,58 (t, J = 8,0 Hz, 1H) ; 7,90 (d, J = 2,5 Hz, 1H) ; 8,13 (d large, J = 8,0 Hz, 1H) ; 8,38 (s, 1H) ; 8,42 (s large, 1H) ; 10,55 (s, 1H). Spectre de masse (UPLC-MS-DAD-ELSD) : m/z 349 [M+H]⁺. |

Les composés selon l'invention ont fait l'objet d'essais pharmacologiques permettant de déterminer leur effet modulateur sur NOT

### Evaluation de l'activité in vitro sur cellules N2A

Sur une lignée cellulaire (N2A) exprimant de manière endogène le récepteur de souris Nurrl et transfectées de manière stable avec l'élément de réponse liant NOT (NBRE) couplé au gène rapporteur luciférase. Les EC₅₀ sont comprises entre 0,01 et 1000 nM. Les essais ont été réalisés selon le mode opératoire décrit ci dessous.

La lignée cellulaire Neuro-2A provient de source commerciale standard (ATCC). Le clone Neuro-2A a été obtenu à partir d'une tumeur spontannée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4.5 g/L de glucose et 0.4 mg/ml de Généticine. Après une semaine de culture les cellules sont récupérées par de la trypsine 0.25% pendant 30 secondes puis remises en suspension dans du DMEM sans rouge de phenol contenant 4.5g/L de glucose, 10% de sérum délipidé Hyclone et déposées dans des plaques blanches 96 puits fond transparent. Les cellules sont déposées à raison de 60.000 par puit dans 75 µL pendant 24 heures avant l'addition des produits. Les produits sont appliqués dans 25µL et incubés 24 heures supplémentaires. Le jour de la mesure, on ajoute à chaque puit un volume équivalent (100µL) de Steadylite, puis attendre 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0.625% final de DMSO.

Par exemple, les composés n° 4, 7, 19, 29, 32, 43, 58, 67 et 70 ont montré une EC₅₀ de respectivement 0,66 nM, 0,9 nM, 0,6 nM, 1,3 nM, 0,06 nM, 0,3 nM, 1,3 nM, 0,7 et 0,16 nM.

### Evaluation de la liaison au récepteur humain NOT

La liaison directe entre des composés de l'invention et le récepteur humain NOT a été évaluée en utilisant la technologie SPR (surface plasmon resonance). Dans cet essai la protéine est immobilisée de façon covalente à la matrice et la molécule à étudier est injectée dans la chambre contenant la sensor chip. Le signal est directement proportionnel à la quantité de produit fixé à la protéine. Les essais de liaison ont été réalisés dans un instrument BIACORE S51 (Biacore Inc., Piscataway N.J.). La protéine entière GST-NOT (NOT-FL) a été fournie par Invitrogen (PV3265). Le domaine de liaison au ligand de NOT (His-Thr-NOT 329-598) a été exprimé et purifié comme décrit dans Nature 423, 555-560. Les deux protéines, diluées à une concentration de 20µg/ml dans un tampon acétate pH 5.0 contenant 5 mM de DTT, ont été immobilisées sur une surface de carboxymethyl 5' dextrane (CM5 sensor chip, Biacore Inc.) par couplage amine en suivant le protocole recommandé par Biacore en éluant par un tampon HBS-N (10 mM HEPES, 0.15 M NaCl, 3 mM EDTA, pH 7.4). Approximativement 10000-15000 unités de resonance (RU) des protéines sont capturées sur la surface du sensor chip CM5. Les solutions stock des composés à étudier à 1,5 mM dans le DMSO sont diluées en série dans du tampon d'élution (50 mM HEPES pH8 ; 150 mM NaCl ; 10 mM MgCl₂; 2% DMSO, 1 mM DTT) à des concentrations allant de 3,75 à 0,1 µM. Chaque concentration de produit est injectée à 4°C pendant 1 minute à 30 µl/min. La dissociation a été enregistrée pendant 5 minutes sans autre procédure de régénération de la surface. Les signaux obtenus sont corrigés en testant chaque concentration de produit sur une surface de dextrane non modifiée (blanc). Le signal dû au tampon de migration est déduit du signal total (« double referencing ») ainsi que l'effet du DMSO. L'analyse des signaux est effectuée à l'aide du logiciel d'analyse Biacore S51 (version 1.2.1). Les composés sont ensuite classés en fonction de leur niveau de fixation maximal et de paramètres cinétiques de liaison à la protéine immobilisée.

A titre d'exemple, les composés n° 19 et 7 ont une affinité respectivement faible et moyenne.

Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire), la sclérose en plaque ; les traumatismes cérébraux comme l'ischémie et les traumatismes crâniens et l'épilepsie ; les maladies psychiatriques comme la schizophrénie, la dépression, la dépendance à une substance les troubles déficit de l'attention et de l'hyperactivité ; les maladies inflammatoires comme les pathologies vasculaires, l'athérosclérose, les inflammations des articulations, l'arthrose, l'arthrite rhumatoïde ostéoarthrite, maladies inflammatoires allergiques telle que l'asthme et pour finir le traitement de l'ostéoporose, les cancers.

Ces composés pourraient être aussi utilisés comme traitement associés à des greffes et/ou transplantations de cellules souches
Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement.

## Revendications

1. Composés répondant à la formule (I) : 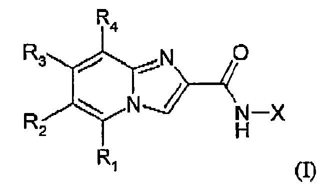 dans laquelle :
X représente l'un des groupes suivants :
. un groupe phényle éventuellement substitué par un ou plusieurs groupes choisis indépendamment les uns des autres parmi les atomes ou groupes suivants : halogène, (C₁- C₆)alcoxy, (C₁-C₆)alkyle, (C₃-C₇)cycloalkyle(C₁-C₆)alkyle, (C₃-C₇)cycloalkyle(C₁₋C₆)alcoxy, NRaRb,
R₁ représente un atome d'hydrogène, un halogène, un groupe (C₁-C₆)alcoxy, un groupe (C₁-C₆)alkyle, un groupe (C₃-C₇)cycloalkyle(C₁-C₆)alkyle, un groupe (C₃-C₇)cycloalkyle(C₁-C₆)alcoxy, un amino, un groupe NRcRd ; les groupes alkyles et alcoxy pouvant éventuellement être substitués par un ou plusieurs halogène, hydroxy, amino, ou groupe (C₁-C₆)alcoxy,
R₂ représente l'un des groupes suivants ;
. un atome d'hydrogène,
. un groupe (C₁-C₆)alkyle éventuellement substitué par un ou plusieurs groupes choisis indépendamment les uns des autres parmi un hydroxy, un halogène, un hydroxy, un amino, un groupe NRaRb, un groupe phényle
. un groupe (C₁-C₆)alcoxy éventuellement substitué par un ou plusieurs groupes choisis indépendamment les uns des autres parmi hydroxy, halogène, hydroxy, amino, groupe NRaRb,
. un groupe (C₃-C₇)cycloalkyle(C₁-C₆)alkyle,
. un groupe (C₃-C₇)cycloalkyle(C₁-C₆)alcoxy,
. un groupe (C₂-C₆)alcényle,
. un groupe (C₂-C₆)alcynyle,
. un groupe -CO-R₅
. un groupe -CO-NR₆R₇
. un groupe -CO-O-R₈
. un groupe -NR₉-CO-R₁₀
. un groupe -NR₁₁R₁₂,
. un atome d'halogène,
. un groupe cyano,
. un groupe phényle éventuellement substitué par un ou plusieurs groupes choisis indépendamment les uns des autres parmi les atomes ou groupes suivantes : halogène, (C₁- C₆)alcoxy, NRaRb, -CO-R₅, -CO-NR₆R₇, -CO-O-R₈, (C₃-C₇)cycloalkyle(C₁-C₆)alkyle, (C₃- C₇)cycloalkyle(C₁-C₆)alcoxy, un groupe (C₁-C₆)alkyle éventuellement substitué par un ou plusieurs hydroxy ou NRaRb,
R₃ représente un atome d'hydrogène, un groupe (C₁-C₆)alkyle, un groupe (C₁-C₆)alcoxy ou un atome d'halogène,
R₄ représente un atome d'hydrogène, un groupe (C₁-C₄)alkyle, un groupe (C₁-C₄)alcoxy ou un atome de fluor,
R₅ représente un atome d'hydrogène, un groupe phényle ou un groupe (C₁-C₆)alkyle,
R₆ et R₇, identiques ou différents, représentent un atome d'hydrogène ou un groupe (C₁-C₆)alkyle ou forment avec l'atome d'azote un cycle de 4 à 7 chaînons incluant éventuellement un autre hétéroatome choisi parmi N, O ou S,
R₈ représente un groupe (C₁-C₆)alkyle,
R₉ et R₁₀, identiques ou différents, représentent un atome d'hydrogène ou un groupe (C₁-C₆)alkyle,
R₁₁ et R₁₂, identiques ou différents, représentent un groupe (C₁-C₆)alkyle ou forment avec l'atome d'azote un cycle de 4 à 7 chaînons incluant éventuellement un autre hétéroatome choisi parmi N, O ou S,
Ra et Rb sont indépendamment l'un de l'autre, hydrogène, (C₁-C₆)alkyle ou forment avec l'atome d'azote un cycle de 4 à 7 chaînons,
Rc est hydrogène et Rd est (C₁-C₆)alkyle
et l'un au moins des substituants R₁, R₂, R₃ et R₄ est différent d'hydrogène ;
et quand R₃ est un méthyle, X est non substitué ;
quand R₁ est un méthyle, X est non substitué
quand R₂ est chlore, X n'est pas un para-fluoro-phényle
à l'état de base ou de sel d'addition à un acide.

2. Composé de formule (I) selon la revendication 1, **caractérisé en ce que** les composés de formule (I) pour lesquels : X est un groupe phényle
à l'état de base ou de sel d'addition à un acide.

3. Composé de formule (I) selon la revendication 1, **caractérisé en ce que** les composés de formule (I) pour lesquels : R₁, R₃ et R₄ sont des atomes d'hydrogène
à l'état de base ou de sel d'addition à un acide.

4. Composés
6-Chloro*-N-*phénylimidazo[1,2*-a*]pyridine-2-carboxamide
8-Méthyl*-N-*phénylimidazo[1,2*-a*]pyridine-2-carboxamide
6-(Diméthylamino)*-N-*phénylimidazo[1,2*-a*]pyridine-2-carboxamide
6-(1-Hydroxy-1-méthyléthyl)*-N-*phénylimidazo[1,2*-a*]pyridine-2-carboxamide
*N-*(4-Fluorophényl)-6-isopropénylimidazo[1,2*-a*]pyridine-2-carboxamide
6-Chloro*-N-*(2-chlorophényl)imidazo[1,2*-a*]pyridine-2-carboxamide
*N*,6-Diphénylimidazo[1,2*-a*]pyridine-2-carboxamide
*N-*Phényl-6-vinylimidazo[1,2*-a*]pyridine-2-carboxamide
6-Ethyl*-N-*phénylimidazo[1,2*-a*]pyridine-2-carboxamide
6-Formyl*-N-*phénylimidazo[1,2*-a*]pyridine-2-carboxamide
6-Ethynyl*-N-*phénylimidazo[1,2*-a*]pyridine-2-carboxamide
6-[3-(1-Hydroxy-1-méthyléthyl)phényl]*-N-*phénylimidazo[1,2*-a*]pyridine-2-carboxamide
6-[Hydroxy(phényl)méthyl]*-N-*phénylimidazo[1,2-]pyridine-2-carboxamide
Chlorhydrate (1:1) de 6-acétyl*-N-*phénylimidazo[1,2*-a*]pyridine-2-carboxamide
6,Isopropyl*-N-*phénylimidazo[1,2*-a*]pyridine-2-carboxamide
6-(1-Hydroxyéthyl)*-N-*phénylimidazo[1,2-*a*]pyridine-2-carboxamide
6-Acétamido*-N-*phénylimidazo[1,2*-a*]pyridine-2-carboxamide
6-(Diméthylamino)-5-méthyl*-N-*phénylimidazo[1,2*-a*]pyridine-2-carboxamide
6-Méthyl*-N-*phénylimidazo[1,2*-a*]pyridine-2-carboxamide
5-Méthyl*-N-*phénylimidazo[1,2*-a*]pyridine-2-carboxamide
7-Méthyl*-N-*phénylimidazo[1,2*-a*]pyridine-2-carboxamide
6-Bromo*-N-*phénylimidazo[1,2*-a*]pyridine-2-carboxamide
6-Fluoro*-N-*phénylimidazo[1,2*-a*]pyridine-2-carboxamide
6,8-Difluoro*-N-*phénylimidazo[1,2*-a*]pyridine-2-carboxamide
6-Bromo-5-méthyl*-N-*phénylimidazo[1,2*-a*]pyridine-2-carboxamide
6-Iodo*-N-*phénylimidazo[1,2*-a*]pyridine-2-carboxamide
6-Cyano*-N-*phénylimidazo[1,2*-a*]pyridine-2-carboxamide
6-(Hydroxyméthyl)*-N-*phénylimidazo[1,2*-a*]pyridine-2-carboxamide
6-Méthoxy*-N-*phénylimidazo[1,2*-a*]pyridine-2-carboxamide
*N*-(4-fluorophényl)-6-(1-hydroxy-1-méthyléthyl)imidazo[1,2*-a*]pyridine-2-carboxamide
6-Benzoyl*-N-*phénylimidazo[1,2*-a*]pyridine-2-carboxamide
6-Isopropényl*-N-*phénylimidazo[1,2*-a*]pyridine-2-carboxamide
6-Chloro*-N-*(3-fluorophényl)imidazo[1,2*-a*]pyridine-2-carboxamide
6-Chloro*-N-*(3-chlorophényl)imidazo[1,2*-a*]pyridine-2-caxboxamide
6-Chloro*-N-*(3-méthoxyphényl)imidazo[1,2*-a*]pyridine-2-carboxamide
6-Chloro*-N-*[4-(diméthylamino)phényl]imidazo[1,2*-a*]pyridine-2-carboxamide
6-Chloro*-N-*(4-chlorophényl)imidazo[1,2*-a*]pyridine-2-carboxamide
6-[2-(Hydroxyméthyl)phényl]*-N-*phénylimidazo[1,2*-a*]pyridine-2-carboxamide
6-[3-(Hydroxyméthyl)phényl]*-N-*phénylimidazo[1,2*-a*]pyridine-2-carboxamide
6-[4-(Hydroxyméthyl)phényl]*-N-*phénylimidazo[1,2*-a*]pyridine-2-carboxamide
6-(2-Formylphényl)*-N-*phénylimidazo[1,2*-a*]pyridine-2-carboxamide
6-(3-Formylphényl)*-N-*phénylimidazo[1,2*-a*]pyridine-2-carboxamide
5,6-Diméthyl*-N-*phénylimidazo[1,2*-a*]pyridine-2-carboxamide
3-[2-(Anilinocarbonyl)imidazo[1,2*-a*]pyridin-6-yl]benzoate de méthyle
6-(3-Acétylphényl)*-N-*phénylimidazo[1,2*-a*]pyridine-2-carboxamide
6-(3-Fluorophényl)*-N-*phénylimidazo[1,2*-a*]pyridine-2-carboxamide
6-(4-Méthylphényl)*-N-*phénylimidazo[1,2*-a*]pyridine-2-carboxamide
6-(3-Méthoxyphényl)*-N-*phénylimidazo[1,2*-a*]pyridine-2-carboxamide
6-[3-(Aminométhyl)phényl]*-N-*phénylimidazo[1,2*-a*]pyridine-2-carboxamide
6-(3-Chlorophényl)*-N-*phénylimidazo[1,2*-a*]pyridine-2-carboxamide
6-(3-Carbamoylphényl)*-N-*phénylimidazo[1,2*-a*]pyridine-2-carboxamide
6-[3-(1-Hydroxyéthyl)phényl]*-N-*phénylimidazo[1,2*-a*]pyridine-2-carboxamide
6-(3-Méthylphényl)*-N-*phénylimidazo[1,2*-a*]pyridine-2-carboxamide
6-(Diéthylamino)*-N-*phénylimidazo[1,2*-a*]pyridine-2-carboxamide et son chlorhydrate (1:1)
6-[3-(Méthylcarbamoyl)phényl]*-N-*phenylimidazo[1,2*-a*]pyridine-2-carboxamide et son chlorhydrate (1:1)
6-Carbamoyl*-N-*phénylimidazo[1,2*-a*]pyridine-2-carboxamide
6-(Diméthylamino)*-N-*(3-fluorophényl)imidazo[1,2*-*a]pyridine-2-carboxamide
*N*-(2,5-difluorophényl)-6-(diméthylamino)imidazo[1,2*-*a]pyridine-2-carboxamide
*N*-(2,3-difluorophényl)-6-(diméthylamino)imidazo[1,2*-*a]pyridine-2-carboxamide
6-(Dimétlaylamino)*-N-*(2-fluorophényl)imidazo[1,2*-*a]pyridine-2-carboxamide
*N*-(3-fluorophényl)-6-[3-(hydroxyméthyl)phényl]imidazo[1,2*-*a]pyridine-2-carboxamide
*N*-(3,5-difluorophényl)-6-[3-(hydroxyméthyl)phényl]imidazo[1,2*-*a]pyridine-2-carboxamide
*N*-(2-chlorophényl)-6-[3-(hydroxyméthyl)phényl]imidazo[1,2-a]pyridine-2-carboxamide
6-[3-(hydroxyméthyl)phényl]*-N-*(3-méthylphényl)imidazo[1,2-a]pyridine-2-carboxamide
*N*-(2,5-difluorophényl)-6-[3-(hydroxyméthyl)phényl]imidazo[1,2-a]pyridine-2-carboxamide
*N*-(2,3-difluorophényl)-6-[3-(hydroxyméthyl)phényl]imidazo[1,2-a]pyridine-2-carboxamide
*N*-(2-fluorophényl)-6-[3-(hydroxyméthyl)phényl]imidazo[1,2-a]pyridine-2-carboxamide
*N*-(5-chloro-2-fluorophényl)-6-[3-(hydroxyméthyl)phényl]imidazo[1,2-a]pyridine-2-carboxamide
6-Morpholin-4-yl*-N-*phénylimidazo[1,2*-a*]pyridine-2-carboxamide et son chlorhydrate (1:1)
6-Azétidin-1-yl*-N-*phénylimidazo[1,2*-a*]pyridine-2-carboxamide
6-Iodo-5-méthyl*-N-*phénylimidazo[1,2*-a*]pyridine-2-carboxamide
*N*-(3,5-difluorophényl)-6-iodoimidazo[1,2-a]pyridine-2-carboxamide
*N-*(3-chlorophényl)-6-[3-(hydroxyméthyl)phényl]imidazo[1,2-a]pyridine-2-carboxamide
*N-*(3,5-difluorophényl)-6-(diméthylamino)imidazo[1,2-a]pyridine-2-carboxamide
*N-*(2-chlorophényl)-6-(diméthylamino)imidazo[1,2,a]pyridine-2-carboxamide
6-(Diméthylamino)*-N-*[3-(trifluorométhoxy)phényl]imidazo[1,2,a]pyridine-2-carboxamide
*N-*(3-chloro-2-fluorophényl)-6-[3-(hydroxyméthyl)phényl]imidazo[1,2-a]pyridine-2-carboxamide
6-(Diméthylamino)*-N-*(3-méthylphényl)imidazo[1,2-a]pyridine-2-carboxamide
*N-*(3-chlorophényl)-6-(diméthylamino)imidazo[1,2-a]pyridine-2-carboxamide
*N-*(5-chloro-2-fluorophényl)-6-(diméthylamino)imidazo[1,2-a]pyridine-2-carboxamide
*N-*(3-chloro-2-fluorophényl)-6-(diméthylamino)imidazo[1,2-a]pyridine-2-carboxamide
*N-*[3-(difluorométhoxy)phényl]-6-(diméthylamino)imidazo[1,2-a]pyridine-2-carboxamide
6-(Diméthylamino)*-N-*[3-(trifluorométhyl)phényl]imidazo[1,2-a]pyridine-2-carboxamide
à l'état de base ou de sel d'addition à un acide.

5. Médicament, **caractérisé en ce qu'**il comprend un composé de formule (I) selon l'une quelconque des revendications 1 à 4, ou un sel d'addition de ce composé à un acide pharmaceutiquement acceptable.

6. Composition pharmaceutique, **caractérisée en ce qu'**elle comprend un composé de formule (I) selon l'une quelconque des revendications 1 à 4, ou un sel pharmaceutiquement acceptable, de ce composé, ainsi qu'au moins un excipient pharmaceutiquement acceptable.

7. Composé de formule (I) selon l'une quelconque des revendications 1 à 4 pour utilisation pour le traitement et la prévention des maladies neurodégénératives.

8. Composé de formule (I) selon l'une quelconque des revendications 1 à 4 pour utilisation pour le traitement et la prévention de la sclérose en plaque ; des traumatismes cérébraux et de l'épilepsie.

9. Composé de formule (I) selon l'une quelconque des revendications 1 à 4 pour utilisation pour le traitement et la prévention des maladies psychiatriques.

10. Composé de formule (I) selon l'une quelconque des revendications 1 à 4 pour utilisation pour le traitement et la prévention des maladies inflammatoires.

11. Composé de formule (I) selon l'une quelconque des revendications 1 à 4 pour utilisation pour le traitement et la prévention de l'ostéoporose et les cancers.

12. Composé de formule (I) selon l'une quelconque des revendications 1 à 4 pour utilisation pour le traitement et la prévention de la maladie de Parkinson, d'Alzheimer, des tauopathies.

13. Composé de formule (I) selon l'une quelconque des revendications 1 à 4 pour utilisation pour le traitement et la prévention de la schizophrénie, la dépression, la dépendance à une substance les troubles déficit de l'attention et de l'hyperactivité.

## Claims

1. Compounds corresponding to the formula (I): 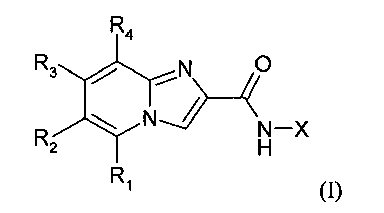 in which:
X represents one of the following groups:
. a phenyl group optionally substituted by one or more groups chosen, independently of one another, from the following atoms or groups: halogen, (C₁-C₆)alkoxy, (C₁-C₆)alkyl, (C₃-C₇)cycloalkyl(C₁-C₆)alkyl, (C₃-C₇)cycloalkyl(C₁-C₆)alkoxy or NRaRb,
R₁ represents a hydrogen atom, a halogen, a (C₁-C₆)alkoxy group, a (C₁-C₆)alkyl group, a (C₃-C₇)cycloalkyl(C₁-C₆)alkyl group, a (C₃-C₇)cycloalkyl(C₁-C₆)alkoxy group, an amino or an NRcRd group; it being possible for the alkyl and alkoxy groups optionally to be substituted by one or more halogen, hydroxyl, amino, or (C₁-C₆)alkoxy group,
R₂ represents one of the following groups:
. a hydrogen atom,
. a (C₁-C₆)alkyl group optionally substituted by one or more groups chosen, independently of one another, from a hydroxyl, a halogen, an amino, an NRaRb group or a phenyl group,
. a (C₁-C₆)alkoxy group optionally substituted by one or more groups chosen, independently of one another, from hydroxyl, halogen, amino or an NRaRb group,
. a (C₃-C₇)cycloalkyl(C₁-C₆)alkyl group,
. a (C₃-C₇)cycloalkyl(C₁-C₆)alkoxy group,
. a (C₂-C₆)alkenyl group,
. a (C₂-C₆)alkynyl group,
. a -CO-R₅ group,
. a -CO-NR₆R₇ group,
. a -CO-O-R₈ group,
. a -NR₉-CO-R₁₀ group,
. a -NR₁₁R₁₂ group,
. a halogen atom,
. a cyano group,
. a phenyl group optionally substituted by one or more groups chosen, independently of one another, from the following atoms or groups: halogen, (C₁-C₆)alkoxy, NRaRb, -CO-R₅, -CO-NR₆R₇, -CO-O-R₈, (C₃-C₇)- cycloalkyl(C₁-C₆)alkyl, (C₃-C₇)cycloalkyl(C₁-C₆)alkoxy, a (C₁-C₆)alkyl group optionally substituted by one or more hydroxyl or NRaRb,
R₃ represents a hydrogen atom, a (C₁-C₆)alkyl group, a (C₁-C₆)alkoxy group or a halogen atom,
R₄ represents a hydrogen atom, a (C₁-C₄)alkyl group, a (C₁-C₄)alkoxy group or a fluorine atom,
R₅ represents a hydrogen atom, a phenyl group or a (C₁-C₆)alkyl group,
R₆ and R₇, which are identical or different, represent a hydrogen atom or a (C₁-C₆)alkyl group or form, with the nitrogen atom, a 4- to 7-membered ring optionally including another heteroatom chosen from N, O or S,
R₈ represents a (C₁-C₆)alkyl group,
R₉ and R₁₀, which are identical or different, represent a hydrogen atom or a (C₁-C₆)alkyl group,
R₁₁ and R₁₂, which are identical or different, represent a (C₁-C₆)alkyl group or form, with the nitrogen atom, a 4- to 7-membered ring optionally including another heteroatom chosen from N, O or S,
Ra and Rb are, independently of one another, hydrogen or (C₁-C₆)alkyl or form, with the nitrogen atom, a 4- to 7-membered ring,
Rc is hydrogen and Rd is (C₁-C₆)alkyl,
and at least one of the R₁, R₂, R₃ and R₄ substituents is other than hydrogen;
and, when R₃ is a methyl, X is unsubstituted;
when R₁ is a methyl, X is unsubstituted;
when R₂ is chlorine, X is not a para-fluorophenyl;
in the form of the base or of an addition salt with an acid.

2. Compound of formula (I) according to Claim 1, **characterized in that** the compounds of formula (I) for which: X is a phenyl group
in the form of the base or of an addition salt with an acid.

3. Compound of formula (I) according to Claim 1, **characterized in that** the compounds of formula (I) for which: R₁, R₃ and R₄ are hydrogen atoms in the form of the base or of an addition salt with an acid.

4. Compounds according to Claim 1:
6-Chloro*-N-*phenylimidazo[1,2*-a*]pyridine-2-carboxamide
8-Methyl*-N-*phenylimidazo[1,2*-a*]pyridine-2-carboxamide
6-(Dimethylamino)*-N-*phenylimidazo[1,2*-a*]pyridine-2-carboxamide
6-(1-Hydroxy-1-methylethyl)*-N-*phenylimidazo[1,2*-a*]pyridine-2-carboxamide
*N*-(4-Fluorophenyl)-6-isopropenylimidazo[1,2*-a*]pyridine-2-carboxamide
6-Chloro*-N-*(2-chlorophenyl)imidazo[1,2*-a*]pyridine-2-carboxamide
*N,*6-Diphenylimidazo[1,2*-a*]pyridine-2-carboxamide
*N-*Phenyl-6-vinylimidazo[1,2*-a*]pyridine-2-carboxamide
6-Ethyl*-N-*phenylimidazo[1,2*-a*]pyridine-2-carboxamide
6-Formyl*-N-*phenylimidazo[1,2*-a*]pyridine-2-carboxamide
6-Ethynyl*-N-*phenylimidazo[1,2*-a*]pyridine-2-carboxamide
6-[3-(1-Hydroxy-1-methylethyl)phenyl]*-N-*phenylimidazo[1,2*-a*]pyridine-2-carboxamide
6-[Hydroxy(phenyl)methyl]*-N-*phenylimidazo[1,2*-a*]pyridine-2-carboxamide
6-Acetyl*-N-*phenylimidazo[1,2*-a*]pyridine-2-carboxamide hydrochloride (1:1)
6-Isopropyl*-N-*phenylimidazo[1,2*-a*]pyridine-2-carboxamide
6-(1-Hydroxyethyl)*-N-*phenylimidazo[1,2*-a*]pyridine-2-carboxamide
6-Acetamido*-N-*phenylimidazo[1,2*-a*]pyridine-2-carboxamide
6-(Dimethylamino)-5-methyl*-N-*phenylimidazo[1,2*-a*]pyridine-2-carboxamide
6-Methyl*-N-*phenylimidazo[1,2*-a*]pyridine-2-carboxamide
5-Methyl*-N-*phenylimidazo[1,2*-a*]pyridine-2-carboxamide
7-Methyl*-N-*phenylimidazo[1,2*-a*]pyridine-2-carboxamide
6-Bromo*-N-*phenylimidazo[1,2*-a*]pyridine-2-carboxamide
6-Fluoro*-N-*phenylimidazo[1,2*-a*]pyridine-2-carboxamide
6,8-Difluoro*-N-*phenylimidazo[1,2*-a*]pyridine-2-carboxamide
6-Bromo-5-methyl*-N-*phenylimidazo[1,2*-a*]pyridine-2-carboxamide
6-Iodo*-N-*phenylimidazo[1,2*-a*]pyridine-2-carboxamide
6-Cyano*-N-*phenylimidazo[1,2*-a*]pyridine-2-carboxamide
6-(Hydroxymethyl)*-N-*phenylimidazo[1,2*-a*]pyridine-2-carboxamide 6-Methoxy*-N-*phenylimidazo[1,2*-a*]pyridine-2-carboxamide
*N*-(4-fluorophenyl)-6-(1-hydroxy-1-methylethyl)imidazo[1,2*-a*]pyridine-2-carboxamide
6-Benzoyl*-N-*phenylimidazo[1,2*-a*]pyridine-2-carboxamide
6-Isopropenyl*-N-*phenylimidazo[1,2*-a*]pyridine-2-carboxamide 6-Chloro*-N-*(3-fluorophenyl)imidazo[1,2*-a*]pyridine-2-carboxamide 6-Chloro*-N-*(3-chlorophenyl)imidazo[1,2*-a*]pyridine-2-carboxamide 6-Chloro*-N-*(3-methoxyphenyl)imidazo[1,2*-a*]pyridine-2-carboxamide 6-Chloro*-N-*[4-(dimethylamino)phenyl]imidazo[1,2*-a*]pyridine-2-carboxamide 6-Chloro*-N-*(4-chlorophenyl)imidazo[1,2*-a*]pyridine-2-carboxamide 6-[2-(Hydroxymethyl)phenyl]*-N-*phenylimidazo[1,2*-a*]pyridine-2-carboxamide 6-[3-(Hydroxymethyl)phenyl]*-N-*phenylimidazo[1,2*-a*]pyridine-2-carboxamide 6-[4-(Hydroxymethyl)phenyl]*-N-*phenylimidazo[1,2*-a*]pyridine-2-carboxamide 6-(2-Formylphenyl)*-N-*phenylimidazo[1,2*-a*]pyridine-2-carboxamide 6-(3-Formylphenyl)*-N-*phenylimidazo[1,2*-a*]pyridine-2-carboxamide 5,6-Dimethyl*-N-*phenylimidazo[1,2*-a*]pyridine-2-carboxamide Methyl 3-[2-(anilinocarbonyl)imidazo[1,2*-a*]pyridin-6-yl]benzoate 6-(3-Acetylphenyl)*-N-*phenylimidazo[1,2*-a*]pyridine-2-carboxamide 6-(3-Fluorophenyl)*-N-*phenylimidazo[1,2*-a*]pyridine-2-carboxamide 6-(4-Methylphenyl)*-N-*phenylimidazo[1,2*-a*]pyridine-2-carboxamide 6-(3-Methoxyphenyl)*-N-*phenylimidazo[1,2*-a*]pyridine-2-carboxamide 6-[3-(Aminomethyl)phenyl]*-N-*phenylimidazo[1,2*-a*]pyridine-2-carboxamide 6-(3-Chlorophenyl)*-N-*phenylimidazo[1,2*-a*]pyridine-2-carboxamide 6-(3-Carbamoylphenyl)*-N-*phenylimidazo[1,2*-a*]pyridine-2-carboxamide 6-[3-(1-Hydroxyethyl)phenyl]*-N-*phenylimidazo[1,2*-a*]pyridine-2-carboxamide 6-(3-Methylphenyl)*-N-*phenylimidazo[1,2*-a*]pyridine-2-carboxamide 6-(Diethylamino)*-N-*phenylimidazo[1,2*-a*]pyridine-2-carboxamide and its hydrochloride (1:1)
6-[3-(Methylcarbamoyl)phenyl]*-N-*phenylimidazo[1,2*-a*]pyridine-2-carboxamide and
its hydrochloride (1:1)
6-Carbamoyl*-N-*phenylimidazo[1,2*-a*]pyridine-2-carboxamide 6-(Dimethylamino)*-N-*(3-fluorophenyl)imidazo[1,2*-a*]pyridine-2-carboxamide *N*-(2,5-Difluorophenyl)-6-(dimethylamino)imidazo[1,2*-a*]pyridine-2-carboxamide *N-*(2,3-Difluorophenyl)-6-(dimethylamino)imidazo[1,2*-a*]pyridine-2-carboxamide 6-(Dimethylamino)*-N-*(2-fluorophenyl)imidazo[1,2*-a*]pyridine-2-carboxamide *N-*(3-Fluorophenyl)-6-[3-(hydroxymethyl)phenyl]imidazo[1,2*-a*]pyridine-2-carboxamide
*N-*(3,5-Difluorophenyl)-6-[3-(hydroxymethyl)phenyl]imidazo[1,2*-a*]pyridine-2-carboxamide
*N*-(2-Chlorophenyl)-6-[3-(hydroxymethyl)phenyl]imidazo[1,2*-a*]pyridine-2-carboxamide
6-[3-(Hydroxymethyl)phenyl]*-N-*(3-methylphenyl)imidazo[1,2*-a*]pyridine-2-carboxamide
*N-*(2,5-Difluorophenyl)-6-[3-(hydroxymethyl)phenyl]imidazo[1,2-*a*]pyridine-2-carboxamide
*N-*(2,3-Difluorophenyl)-6-[3-(hydroxymethyl)phenyl]imidazo[1,2*-a*]pyridine-2-carboxamide
*N-*(2-Fluorophenyl)-6-[3-(hydroxymethyl)phenyl]imidazo[1,2*-a*]pyridine-2-carboxamide
*N-*(5-Chloro-2-fluorophenyl)-6-[3-(hydroxymethyl)phenyl]imidazo[1,2*-a*]pyridine-2-carboxamide
6-Morpholin-4-yl*-N-*phenylimidazo[1,2*-a*]pyridine-2-carboxamide and its hydrochloride (1:1)
6-Azetidin-1-yl*-N-*phenylimidazo[1,2*-a*]pyridine-2-carboxamide
6-Iodo-5-methyl*-N-*phenylimidazo[1,2*-a*]pyridine-2-carboxamide
*N-*(3,5-Difluorophenyl)-6-iodoimidazo[1,2*-a*]pyridine-2-carboxamide
*N-*(3-Chlorophenyl)-6-[3-(hydroxymethyl)phenyl]imidazo[1,2*-a*]pyridine-2-carboxamide
*N-*(3,5-Difluorophenyl)-6-(dimethylamino)imidazo[1,2*-a*]pyridine-2-carboxamide
*N-*(2-Chlorophenyl)-6-(dimethylamino)imidazo[1,2*-a*]pyridine-2-carboxamide
6-(Dimethylamino)*-N-*[3-(trifluoromethoxy)phenyl]imidazo[1,2*-a*]pyridine-2-carboxamide
*N-*(3-Chloro-2-fluorophenyl)-6-[3-(hydroxymethyl)phenyl]imidazo[1,2*-a*]pyridine-2-carboxamide
6-(Dimethylamino)*-N-*(3-methylphenyl)imidazo[1,2*-a*]pyridine-2-carboxamide
*N-*(3-Chlorophenyl)-6-(dimethylamino)imidazo[1,2*-a*]pyridine-2-carboxamide
*N-*(5-Chloro-2-fluorophenyl)-6-(dimethylamino)imidazo[1,2*-a*]pyridine-2-carboxamide
*N-*(3-Chloro-2-fluorophenyl)-6-(dimethylamino)imidazo[1,2*-a*]pyridine-2-carboxamide
*N-*[3-(Difluoromethoxy)phenyl]-6-(dimethylamino)imidazo[1,2*-a*]pyridine-2-carboxamide
6-(Dimethylamino)*-N-*[3-(trifluoromethyl)phenyl]imidazo[1,2*-a*]pyridine-2-carboxamide
in the form of the base or of an addition salt with an acid.

5. Medicament, **characterized in that** it comprises a compound of formula (I) according to any one of Claims 1 to 4 or an addition salt of this compound with a pharmaceutically acceptable acid.

6. Pharmaceutical composition, **characterized in that** it comprises a compound of formula (I) according to any one of Claims 1 to 4 or a pharmaceutically acceptable salt of this compound, and also at least one pharmaceutically acceptable excipient.

7. Compound of formula (I) according to any one of Claims 1 to 4 for use for the treatment and prevention of neurodegenerative diseases.

8. Compound of formula (I) according to any one of Claims 1 to 4 for use for the treatment and prevention of multiple sclerosis, cerebral traumas and epilepsy.

9. Compound of formula (I) according to any one of Claims 1 to 4 for use for the treatment and prevention of psychiatric diseases.

10. Compound of formula (I) according to any one of Claims 1 to 4 for use for the treatment and prevention of inflammatory diseases.

11. Compound of formula (I) according to any one of Claims 1 to 4 for use for the treatment and prevention of osteoporosis and cancers.

12. Compound of formula (I) according to any one of Claims 1 to 4 for use for the treatment and prevention of Parkinson's disease, Alzheimer's disease or tauopathies.

13. Compound of formula (I) according to any one of Claims 1 to 4 for use for the treatment and prevention of schizophrenia, depression, substance dependence or attention deficit hyperactivity disorders.

## Patentansprüche

1. Verbindungen der Formel (I): 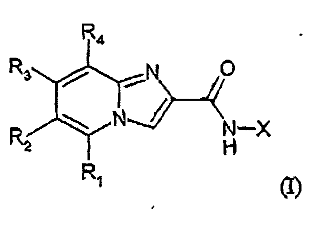 worin
X für eine der folgenden Gruppen steht:
. eine Phenylgruppe, die gegebenenfalls durch eine oder mehrere Gruppen substituiert ist, die unabhängig voneinander unter den folgenden Atomen bzw. Gruppen ausgewählt sind: Halogen, (C₁-C₆) -Alkoxy, (C₁-C₆) -Alkyl, (C₃-C₇) -Cyclo- alkyl- (C₁-C₆) -alkyl, (C₃-C₇) -Cycloalkyl- (C₁-C₆) - alkoxy, und NRaRb,
R₁ für ein Wasserstoffatom, ein Halogen, eine (C₁- C₆)-Alkoxygruppe, eine (C₁-C₆) -Alkylgruppe, eine (C₃-C₇)-Cycloalkyl-(C₁-C₆)-alkylgruppe, eine (C₃- C₇) -Cycloalkyl-(C₁-C₆) -alkoxygruppe, ein Amino oder eine NRcRd-Gruppe steht; wobei die Alkyl- und Alkoxygruppen gegebenenfalls durch ein bzw. eine oder mehrere Halogenatome, Hydroxygruppen, Aminogruppen oder (C₁-C₆)-Alkoxygruppen substituiert sind,
R₂ für eine der folgenden Gruppen steht:
. ein Wasserstoffatom,
. eine (C₁-C₆)-Alkylgruppe, die gegebenenfalls durch ein oder mehrere Atome bzw. eine oder mehrere Gruppen, die unabhängig voneinander unter Hydroxy, Halogen, Hydroxy, Amino, einer NRaRb-Gruppe und einer Phenylgruppe ausgewählt sind, substituiert ist,
. eine (C₁-C₆)-Alkoxygruppe, die gegebenenfalls durch ein oder mehrere Atome bzw. eine oder mehrere Gruppen, die unabhängig voneinander unter Hydroxy, Halogen, Hydroxy, Amino und einer NRaRb-Gruppe ausgewählt sind, substituiert ist,
. eine (C₃-C₇)-Cycloalkyl-(C₁-C₆)-alkylgruppe,
. eine (C₃-C₇)-Cycloalkyl-(C₁-C₆)-alkoxygruppe,
. eine (C₂-C₆)-Alkenylgruppe,
. eine (C₂-C₆)-Alkinylgruppe,
. eine -CO-R₅-Gruppe,
. eine -CO-NR₆R₇-Gruppe,
. eine -CO-O-R₈-Gruppe,
. eine -NR₉-CO-R₁₀-Gruppe,
. eine -NR₁₁R₁₂-Gruppe,
. ein Halogenatom,
. eine Cyanogruppe,
. eine Phenylgruppe, die gegebenenfalls durch eine oder mehrere Gruppen substituiert ist, die unabhängig voneinander unter den folgenden Atomen bzw. Gruppen ausgewählt sind: Halogen, (C₁-C₆) -Alkoxy, NRaRb, -CO-R₅, -CO-NR₆R₇, -CO-O- R₈, (C₃-C₇) -Cycloalkyl- (C₁-C₆) -alkyl, (C₃-C₇) - Cycloalkyl-(C₁-C₆)-alkoxy und einer (C₁-C₆) - Alkylgruppe, die gegebenenfalls durch ein oder mehrere Hydroxy oder NRaRb substituiert ist,
R₃ für ein Wasserstoffatom, eine (C₁-C₆) -Alkyl- gruppe, eine (C₁-C₆)-Alkoxygruppe oder ein Halogenatom steht,
R₄ für ein Wasserstoffatom, eine (C₁-C₄)-Alkyl- gruppe, eine (C₁-C₄) -Alkoxygruppe oder ein Fluoratom steht,
R₅ für ein Wasserstoffatom, eine Phenylgruppe oder eine (C₁-C₆)-Alkylgruppe steht,
R₆ und R₇ gleich oder verschieden sind und für ein Wasserstoffatom oder eine (C₁-C₆)-Alkylgruppe stehen oder mit dem Stickstoffatom einen 4- bis 7-gliedrigen Ring, der gegebenenfalls ein weiteres unter N, O und S ausgewähltes Heteroatom enthält, bilden,
R₈ für eine (C₁-C₆) -Alkylgruppe steht,
R₉ und R₁₀ gleich oder verschieden sind und für ein Wasserstoffatom oder eine (C₁-C₆)-Alkylgruppe stehen,
R₁₁ und R₁₂ gleich oder verschieden sind und für eine (C₁-C₆)-Alkylgruppe stehen oder mit dem Stickstoffatom einen 4- bis 7-gliedrigen Ring, der gegebenenfalls ein weiteres unter N, O und S ausgewähltes Heteroatom enthält, bilden,
Ra und Rb unabhängig voneinander für Wasserstoff oder (C₁-C₆)-Alkyl stehen oder mit dem Stick- stoffatom einen 4- bis 7-gliedrigen Ring bilden,
Rc für Wasserstoff steht und Rd für (C₁-C₆)-Alkyl steht
und mindestens einer der Substituenten R₁, R₂, R₃ und R₄ nicht für Wasserstoff steht;
und wenn R₃ für Methyl steht, X unsubstituiert ist;
wenn R₁ für Methyl steht, X unsubstituiert ist;
wenn R₂ für Chlor steht, X nicht für para-Fluorphenyl steht;
in Basen- oder Säueradditionssalzform.

2. Verbindung der Formel (I) nach Anspruch 1, **dadurch gekennzeichnet, daß** die Verbindungen der Formel (I), für die: X für eine Phenylgruppe steht,
in Basen- oder Säueradditionssalzform.

3. Verbindung der Formel (I) nach Anspruch 1, **dadurch gekennzeichnet, daß** die Verbindungen der Formel (I), für die: R₁, R₃ und R₄ für Wasserstoffatome stehen,
in Basen- oder Säureadditionssalzform.

4. Verbindungen nach Anspruch 1:
6-Chlor*-N-*phenylimidazo[1,2*-a*]pyridin-2-carboxamid
8-Methyl*-N-*phenylimidazo[1,2*-a*]pyridin-2-carboxamid
6-(Dimethylamino)*-N-*phenylimidazo[1,2*-a*]pyridin-2-carboxamid
6-(1-Hydroxy-1-methylethyl)*-N-*phenylimidazo[1,2-a]pyridin-2-carboxamid
*N-*(4-Fluorphenyl)-6-isopropenylimidazo[1,2*-a*]-pyridin-2-carboxamid
6 6-Chlor*-N-*(2-chlorphenyl)imidazo[1,2*-a*]pyridin-2-carboxamid
*N*,6-Diphenylimidazo[1,2*-a*]pyridin-2-carboxamid
*N-*Phenyl-6-vinylimidazo[1,2*-a*]pyridin-2-carboxamid
6-Ethyl*-N-*phenylimidazo[1,2*-a*]pyridin-2-carboxamid
6-Formyl*-N-*phenylimidazo[1,2*-a*]pyridin-2-carboxamid
6-Ethinyl*-N-*phenylimidazo[1,2*-a*]pyridin-2-carboxamid
6-[3-(1-Hydroxy-1-methylethyl)phenyl]*-N-*phenyl-imidazo[1,2-α]pyridin-2-carboxamid
6-[Hydroxy(phenyl)methyl]*-N-*phenylimidazo[1,2*-a*]-pyridin-2-carboxamid
6-Acetyl*-N-*phenylimidazo[1,2*-a*]pyridin-2-carboxamid-Hydrochlorid (1:1)
6-Isopropyl*-N-*phenylimidazo[1,2*-a*]pyridin-2-carboxamid
6-(1-Hydroxyethyl)*-N-*phenylimidazo[1,2*-a*]pyridin-2-carboxamid
6-Acetamido-*N*-phenylimidazo[1,2*-a*]pyridin-2-carboxamid
6-(Dimethylamino)-5-methyl*-N-*phenylimidazo[1,2*-a*]-pyridin-2-carboxamid
6-Methyl*-N-*phenylimidazo[1,2*-a*]pyridin-2-carboxamid
5-Methyl*-N-*phenylimidazo[1,2*-a*]pyridin-2-carboxamid 7-Methyl*-N-*phenylimidazo[1,2*-a*]pyridin-2-carboxamid 6-Brom*-N-*phenylimidazo[1,2*-a*]pyridin-2-carboxamid 6-Fluor*-N-*phenylimidazo[1,2*-a*]pyridin-2-carboxamid 6,8-Difluor*-N-*phenylimidazo[1,2*-a*]pyridin-2-carboxamid 6-Brom-5-methyl*-N-*phenylimidazo[1,2*-a*]pyridin-2-carboxamid 6-Iod*-N-*phenylimidazo[1,2*-a*]pyridin-2-carboxamid 6-Cyano*-N-*phenylimidazo[1,2*-a*]pyridin-2-carboxamid 6-(Hydroxymethyl)*-N-*phenylimidazo[1,2*-a*]pyridin-2-carboxamid 6-Methoxy*-N-*phenylimidazo[1,2*-a*]pyridin-2-carboxamid *N-*(4-Fluorphenyl)-6-(1-hydroxy-1-methylethyl)-imidazo[1,2-α]pyridin-2-carboxamid 6-Benzoyl*-N-*phenylimidazo[1,2*-a*]pyridin-2-carboxamid 6-Isopropenyl*-N-*phenylimidazo[1,2*-a*]pyridin-2-carboxamid 6-Chlor*-N-*(3-fluorphenyl)imidazo[1,2*-a*]pyridin-2-carboxamid 6-Chlor*-N-*(3-chlorphenyl)imidazo[1,2*-a*]pyridin-2-carboxamid 6-Chlor*-N-*(3-methoxyphenyl)imidazo[1,2*-a*]pyridin-2-carboxamid 6-Chlor*-N-*[4-(dimethylamino)phenyl]imidazo[1,2*-a*]-pyridin-2-carboxamid 6-Chlor*-N-*(4-chlorphenyl)imidazo[1,2*-a*]pyridin-2-carboxamid 6-[2-(Hydroxymethyl)phenyl]*-N-*phenylimidazo[1,2-*a*]pyridin-2-carboxamid 6-[3-(Hydroxymethyl)phenyl]*-N-*phenylimidazo[1,2-*a*]pyridin-2-carboxamid 6-[4-(Hydroxymethyl)phenyl]*-N-*phenylimidazo[1,2-a]pyridin-2-carboxamid
6-(2-Formylphenyl)*-N-*phenylimidazo[1,2*-a*]pyridin-2-carboxamid 6-(3-Formylphenyl)*-N-*phenylimidazo[1,2*-a*]pyridin-2-carboxamid 5,6-Dimethyl*-N-*phenylimidazo[1,2*-a*]pyridin-2-carboxamid 3-[2-(Anilinocarbonyl)imidazo[1,2*-a*]pyridin-6-yl]-benzoesäuremethylester 6-(3-Acetylphenyl)*-N-*phenylimidazo[1,2*-a*]pyridin-2-carboxamid 6-(3-Fluorphenyl)*-N-*phenylimidazo[1,2*-a*]pyridin-2-carboxamid 6-(4-Methylphenyl)*-N-*phenylimidazo[1,2*-a*]pyridin-2-carboxamid 6-(3-Methoxyphenyl)*-N-*phenylimidazo[1,2*-a*]pyridin-2-carboxamid 6-[3-(Aminomethyl)phenyl]*-N-*phenylimidazo[1,2*-a*]-pyridin-2-carboxamid 6-(3-Chlorphenyl)*-N-*phenylimidazo[1,2*-a*]pyridin-2-carboxamid 6-(3-Carbamoylphenyl)*-N-*phenylimidazo[1,2*-a*]-pyridin-2-carboxamid 6-[3-(1-Hydroxyethyl)phenyl]*-N-*phenylimidazo[1,2-*a*]pyridin-2-carboxamid 6-(3-Methylphenyl)*-N-*phenylimidazo[1,2*-a*]pyridin-2-carboxamid *6*-(Diethylamino)*-N-*phenylimidazo[1,2*-a*]pyridin-2-carboxamid und dessen Hydrochlorid (1:1) 6-[3-(Methylcarbamoyl)phenyl]*-N-*phenylimidazo[1,2-*a*]pyridin-2-carboxamid und dessen Hydrochlorid (1:1)
6-Carbamoyl*-N-*phenylimidazo[1,2*-a*]pyridin-2-carboxamid 6-(Dimethylamino)*-N-*(3-fluorphenyl)imidazo[1,2*-a*]-pyridin-2-carboxamid *N-*(2,5-Difluorphenyl)-6-(dimethylamino)imidazo-[1,2*-a*]pyridin-2-carboxamid *N-*(2,3-Difluorphenyl)-6-(dimethylamino)imidazo-[1,2*-a*]pyridin-2-carboxamid
6-(Dimethylamino)*-N-*(2-fluorphenyl)imidazo[1,2*-a*]-pyridin-2-carboxamid *N-*(3-Fluorphenyl)-6-[3-(hydroxymethyl)phenyl]-imidazo[1,2-α]pyridin-2-carboxamid *N-*(3,5-Difluorphenyl)-6-[3-(hydroxymethyl)phenyl]-imidazo[1,2-α]pyridin-2-carboxamid *N-*(2-Chlorphenyl)-6-[3-(hydroxymethyl)phenyl]-imidazo[1,2*-a*]pyridin-2-carboxamid 6-[3-(Hydroxymethyl)phenyl]*-N-*(3-methylphenyl)-imidazo[1,2*-a*]pyridin-2-carboxamid *N-*(2,5-Difluorphenyl)-6-[3-(hydroxymethyl)phenyl]-imidazo[1,2-*a*]pyridin-2-carboxamid *N-*(2,3-Difluorphenyl)-6-[3-(hydroxymethyl)phenyl]-imidazo[1,2-α]pyridin-2-carboxamid *N-*(2-Fluorphenyl)-6-[3-(hydroxymethyl)phenyl]-imidazo[1,2-α]pyridin-2-carboxamid *N-*(5-Chlor-2-fluorphenyl)-6-[3-(hydroxymethyl)-phenyl]imidazo[1,2*-a*]pyridin-2-carboxamid 6-Morpholin-4-yl*-N-*phenylimidazo[1,2*-a*]pyridin-2-carboxamid und dessen Hydrochlorid (1:1) 6-Azetidin-1-yl*-N-*phenylimidazo[1,2*-a*]pyridin-2-carboxamid 6-Iod-5-methyl*-N-*phenylimidazo[1,2*-a*]pyridin-2-carboxamid *N-*(3,5-Difluorphenyl)-6-iodoimidazo[1,2*-a*]pyridin-2-carboxamid *N-*(3-Chlorphenyl)-6-[3-(hydroxymethyl)phenyl]-imidazo[1,2-α]pyridin-2-carboxamid *N-*(3,5-Difluorphenyl)-6-(dimethylamino)imidazo-[1,2*-a*]pyridin-2-carboxamid *N-*(2-Chlorphenyl)-6-(dimethylamino)imidazo[1,2*-a*]-pyridin-2-carboxamid 6-(Dimethylamino)*-N-*[3-(trifluormethoxy)phenyl]-imidazo[1,2-α]pyridin-2-carboxamid *N-*(3-Chlor-2-fluorphenyl)-6-[3-(hydroxymethyl)-phenyl]imidazo[1,2*-a*]pyridin-2-carboxamid 6-(Dimethylamino)*-N-*(3-methylphenyl)imidazo[1,2-a]pyridin-2-carboxamid
*N-*(3-Chlorphenyl)-6-(dimethylamino)imidazo[1,2*-a*]-pyridin-2-carboxamid
*N-*(5-Chlor-2-fluorphenyl)-6-(dimethylamino)-imidazo[1,2*-a*]pyridin-2-carboxamid
*N-*(3-Chlor-2-fluorphenyl)-6-(dimethylamino)-imidazo[1,2*-a*]pyridin-2-carboxamid
*N-*[3-(Difluormethoxy)phenyl]-6-(dimethylamino)-imidazo[1,2*-a*]pyridin-2-carboxamid
6-(Dimethylamino)*-N-*[3-(trifluormethyl)phenyl]-imidazo[1,2*-a*]pyridin-2-carboxamid
in Basen- oder Säureadditionssalzform.

5. Medikament, **dadurch gekennzeichnet, daß** es eine Verbindung der Formel (I) nach einem der Ansprüche 1 bis 4 oder ein Additionssalz dieser Verbindung mit einer pharmazeutisch unbedenklichen Säure enthält.

6. Pharmazeutische Zusammensetzung, **dadurch gekennzeichnet, daß** sie eine Verbindung der Formel (I) nach einem der Ansprüche 1 bis 4 oder ein pharmazeutisch unbedenkliches Salz dieser Verbindung sowie mindestens einen pharmazeutisch unbedenklichen Hilfsstoff enthält.

7. Verbindung der Formel (I) nach einem der Ansprüche 1 bis 4 zur Verwendung zur Behandlung und Prävention von neurodegenerativen Erkrankungen.

8. Verbindung der Formel (I) nach einem der Ansprüche 1 bis 4 zur Verwendung zur Behandlung und Prävention von Multipler Sklerose, Hirntraumen und Epilepsie.

9. Verbindung der Formel (I) nach einem der Ansprüche 1 bis 4 zur Verwendung zur Behandlung und Prävention von psychiatrischen Erkrankungen.

10. Verbindung der Formel (I) nach einem der Ansprüche 1 bis 4 zur Verwendung zur Behandlung und Prävention von entzündlichen Erkrankungen.

11. Verbindung der Formel (I) nach einem der Ansprüche 1 bis 4 zur Verwendung zur Behandlung und Prävention von Osteoporose und Krebserkrankungen.

12. Verbindung der Formel (I) nach einem der Ansprüche 1 bis 4 zur Verwendung zur Behandlung und Prävention von Parkinson-Krankheit, Alzheimer-Krankheit und Tauopathien.

13. Verbindung der Formel (I) nach einem der Ansprüche 1 bis 4 zur Verwendung zur Behandlung und Prävention von Schizophrenie, Depression, Substanzabhängigkeit und Aufmerksamkeitsdefizit-Hyperaktivitätssyndrom.
